# EUROPEAN PATENT APPLICATION

(11) **EP 3 572 411 A1**
(43) Date of publication of application: **27.11.2019**
(21) Application number: 18290056.3
(22) Date of filing: 21.05.2018
(51) Int. Cl.: C07D 417/12, C07D 498/04, A61P 31/04, A61K 31/427

(54) **THIAZOLE DERIVATIVES AS METALLO-BETA-LACTAMASE INHIBITORS**

(71) Applicant: Antabio SAS, 31670 Labège (FR)
(72) Inventor: DAVIES, David, Labege 31670 (FR); LEIRIS, Simon, Labege 31670 (FR)
(74) Representative: J A Kemp LLP

(57) **Abstract**

A compound which is a thiazole derivative of Formula (I), or a pharmaceutically acceptable salt thereof, 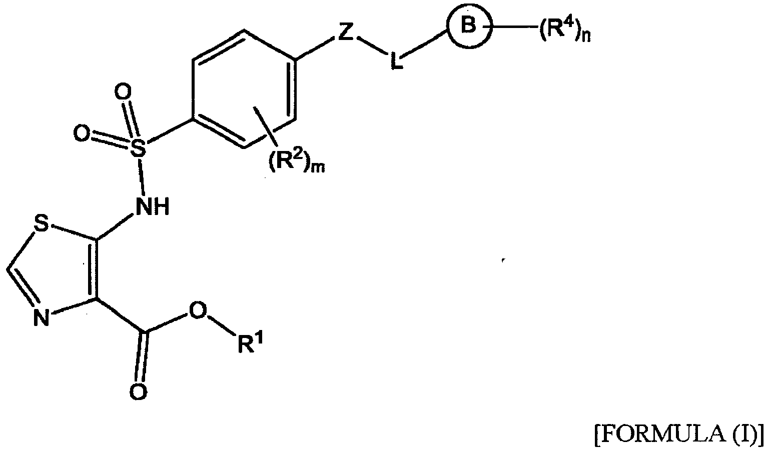 wherein R¹, R², *n,* Z, L, Ring B, R⁴ and *n* are as herein defined. Such compounds are useful in the prevention and treatment of bacterial infection. In particular, the compounds are inhibitors of metallo-β-lactamase and are useful in combination with antibiotic agents such as carbapenems in the treatment of infection caused by bacteria that are resistant to treatment with antibiotic agents when administered alone.

## Description

### Field of the Invention

The present invention relates to compounds which are thiazole derivatives. The compounds of the invention find use in the prevention or treatment of bacterial infection. The invention also provides such compounds *per se* and pharmaceutical compositions comprising such compounds. The compounds of the invention are useful as inhibitors of metallo-β-lactamase (MBL) enzymes. The compounds of the invention can be used in combination therapy, for example in combination with one or more antibiotic agents and optionally with one or more inhibitors of serine-β-lactamase (SBL) enzymes. Such combination therapy has particular applications in prevention or treatment of bacterial infection caused by bacteria which are resistant to treatment by antibiotic agents when administered alone, especially when the resistance is attributable to the presence of metallo-β-lactamase and/or serine-β-lactamase enzymes and treatment with β-lactam antibiotics alone may be unsuccessful. In such cases the combination therapy can rescue the antibacterial activity of the β-lactam antibiotic.

### Background

Bacteria in both clinical and non-clinical settings are becoming increasingly resistant to conventional antibiotics, and this resistance is becoming a serious clinical and epidemiological problem for human health. For example, it has been shown that single amino acid mutations in bacterial DNA-dependent RNA-polymerase can reduce the binding affinity of this target enzyme for antibiotics, leading to a high frequency of resistance (FoR). One approach to addressing FoR that has been previously considered is to develop a single agent that inhibits two related bacterial enzymes. Examples of such agents include gepotidacin, which inhibits two similar DNA-processing components of Topoisomerase II and IV enzymes, (GyrA and ParC) and zoliflodacin, which inhibits two similar ATP-hydrolysing components of Topoisomerase II and IV enzymes (GyrB and ParE). However, this approach is not always suitable for addressing other forms of resistance, for example when microbial resistance to an antibiotic arises through production of a bacterial enzyme able to deactivate the antibacterial drug.

In Gram-negative bacteria, resistance to antibiotics (particularly β-lactam antibiotics) often arises from the production by the organism of β-lactamases. β-Lactamase enzymes include both metallo-β-lactamases (MBL) and serine-β-lactamases (SBL). Serine β-lactamase enzymes use an active serine to hydrolyse β-lactam rings in a covalent mechanism while the structurally different metallo-β-lactamase enzymes use Zn metal coordination and a hydroxide ion to hydrolyse the β-lactam ring. In the field of bacterial β-lactamase enzymes, in particular the Gram-negative area and more particular the Enterobacteriaceae, the older serine-β-lactamase enzymes have been supplemented by the more recently-evolved metallo-β-lactamases. Resistance of gram-negative bacteria to β-lactam antibiotics therefore especially arises from the production by the organism of two types of β-lactamases.

As discussed above, in gram-negative bacteria, resistance to antibiotics often arises from the production by the organism of β-lactamases, especially metallo-β-lactamases (MBL). MBL are resistance determinants of increasing clinical relevance. In fact, because of their broad range, potent carbapenemase activity and resistance to inhibitors, these enzymes can confer resistance to almost all β-lactam antibiotics.

MBLs were first detected in the mid-1960s as carried by mobile DNA elements in species with only low pathogenic potential. However, genes encoding MBL spread among major Gram-negative bacteria during the 1990s and this has led to a health crisis arising from the international dissemination of carbapenem-resistant *Enterobacteriaceae* producing the VIM-type and NDM-type metallo-β-lactamases.

Functional features of these *Enterobacteriaceae* include potent carbapenemase activity and resistance to clinical β-lactamase inhibitors (clavulanate and sulfones). The activity against β-lactams differs between the different metallo-β-lactamases, and substrate specificity might vary from a narrow range (eg, the CphA metallo-β-lactamase of *Aeromonas hydrophila*), to an extended range (eg, the VIM-type metallo-β-lactamases, which can degrade almost all classes of β-lactams apart from the monobactams).

There are three major structural subclasses of MBL which share substantial internal diversity. Members of the different subclasses differ not only in their high degree of sequence diversity, but also in the structure of their active sites. In enzymes of subclasses B1 and B3, the active site contains two zinc ions; in members of subclass B2, the active site contains only one zinc ion.

Acquired metallo-β-lactamases have been detected in strains of *Enterobacteriaceae, Pseudomonas aeruginosa, Acinetobacter baumannii,* and other Gram-negative bacteria. Among acquired MBL, almost all the enzymes belong to subclass B1, which indicates an overall higher propensity for members of this subclass to be captured and spread with mobile genetic elements than for members of subclasses B2 and B3.

As an example, the subclass B1 comprises the IMP-type, the VIM-type, and the NDM-type enzymes.

The IMP-type enzymes, including IMP-1, were first detected in Japan in the late 1980s, and have since been reported worldwide in *Enterobacteriaceae* and in Gram-negative bacteria. The IMP-type enzymes have broad substrate specificity with a high affinity for cephalosporins and carbapenems, but they have little activity against Temocillin.

The VIM-type enzymes, including VIM-2, were first discovered in Europe in the late 1990s and have since been reported worldwide. VIM-type enzymes were initially detected in *P. aeruginosa* and in other Gram-negative bacteria, but have since emerged in *Enterobacteriaceae,* and have become a major problem in some settings. More than 20 different VIM allotypes are known, each with a defined geographical distribution except for VIM-1 and VIM-2, which share a broader distribution than the IMP-type enzymes. The VIM-type metallo-β-lactamases show even broader substrate specificities than the IMP-types, being able to hydrolyse 6-α-methoxy-penicillins. Furthermore, the VIM-type enzymes are unique in the metallo-β-lactamases in that they have a high affinity for carbapenems.

New Delhi metallo-β-lactamase 1 (NDM-1) is a novel metallo-β-lactamase identified initially in a patient hospitalized in New Delhi with an infection caused by *Klebsiella pneumoniae.* Subsequently, organisms in the *Enterobacteriaceae* family containing this new β-lactamase have been found widely distributed throughout India, Pakistan, and Bangladesh and are now occurring in the United Kingdom and in many other countries. The New Delhi metallo-β-lactamase 1 (NDM-1) is a polypeptide of 158 amino acids in length (Accession number AB571289) capable of hydrolyzing a wide range of β-lactam antibiotics including penicillins, cephalosporins and carbapenem antibiotics that are a mainstay for the treatment of antibioticresistant bacterial infections.

Accordingly, there is an urgent need for new antibacterial compounds and compositions and adjuvant therapies for treating bacterial infection, in particular bacterial infection caused by bacteria which express MBL enzymes. There is also an urgent need for new compositions for treating bacterial infection by bacteria which exhibit high resistance, particularly when resistance to antibiotic agents (especially β-lactam antibiotic agents) arises through production by the bacteria of one or more enzyme able to deactivate the antibacterial drug. The present invention aims to address some or all of these issues.

### Summary of the Invention

Previously, the inventors reported in WO 2014/198849 that certain thiazole derivatives are inhibitors of metallo-β-lactamases, including NDM-1. The inventors have now surprisingly found that compounds of Formula (I) are potent inhibitors of metallo-β-lactamases, including NDM-1, and have improved properties compared to the compounds disclosed in WO 2014/198849. The compounds therefore are useful in treating and preventing bacterial infection, for example by use in combination with β-lactam antibiotics.

The inventors have also found that the compounds of Formula (I) can advantageously be used in combination with inhibitors of serine-β-lactamase enzymes and other antibiotic agents such as β-lactam antibiotics e.g. carbapenem antibiotics. Such combination therapies have particular relevance in the prevention or treatment of bacterial infection caused by bacteria which exhibit a high degree of resistance to the antibiotic agents when administered alone, especially when the bacterial infection is caused by bacteria which produce β-lactamase enzymes.

Accordingly, the invention provides a compound as set out in aspect [1] below.

The present invention also provides a pharmaceutical composition comprising a compound as described herein and together with at least one pharmaceutically acceptable carrier or diluent and optionally (i) an antibiotic agent and/or (ii) a serine-β-lactamase inhibitor. Also provided is a combination of a compound as described herein and (i) an antibiotic agent and/or (ii) a serine-β-lactamase inhibitor.

The compounds, compositions and combinations of the invention are particularly useful in the treatment or prevention of bacterial infection caused by bacteria which are resistant to treatment by the antibiotic agent when administered alone, and especially when the resistance is attributable to the presence of metallo-β-lactamase and/or serine-β-lactamase enzymes. In patients suffering from or susceptible to infection by such bacteria, treatment with β-lactam antibiotics alone may be unsuccessful.

The present invention, in particular, provides:
1. A compound which is a thiazole derivative of Formula (I), or a pharmaceutically acceptable salt thereof, 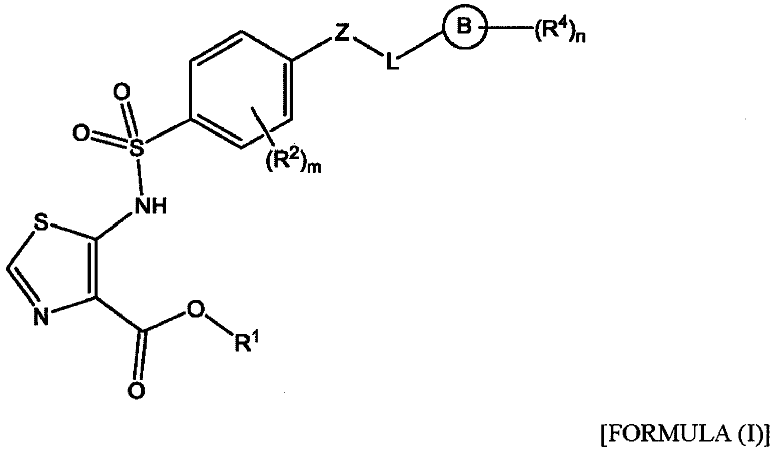 wherein
   ∘ R¹ is selected from:
      - H, R^{1a} and -CH₂OC(O)R^{1a}, wherein R^{1a} is selected from an unsubstituted C₁ to C₄ alkyl group and phenyl; or
      - where the compound of Formula (I) contains a positively charged nitrogen atom, R¹ maybe absent, such that a COO⁻ group is present and the compound forms a zwitterion;
   ∘ each R² is independently selected from halo, R⁸, C₁₋₃ alkyl, O(C₁₋₃ alkyl) and S(C₁₋₃ alkyl), wherein the C₁₋₃ alkyl, O(C₁₋₃ alkyl) and S(C₁₋₃ alkyl) groups are unsubstituted or substituted with 1, 2 or 3 halo substituents and/or one R⁸ substituent, wherein each R⁸ is independently selected from CN and OH;
   ∘ *m* is 0, 1, 2 or 3
   ∘ Z is selected from -NR¹⁰C(O)-, -C(O)NR¹⁰-, -NR¹⁰C(O)NR¹¹-, -NR¹⁰C(O)O- and -OC(O)NR¹⁰;
   ∘ L is selected from C₁₋₄ alkylene, C₃₋₄ alkenylene, C₃₋₄ alkynylene, wherein L is unsubstituted or is substituted with 1 or 2 substituents selected from halogen, -OR¹⁰, -NR¹⁰R¹¹ and -N⁺R¹⁰R¹¹R¹²;
   ∘ Ring B is a five-membered heteroaromatic ring containing 1, 2, 3 or 4 nitrogen atoms, wherein ring B is joined to L via a ring nitrogen atom;
   ∘ each R⁴ is independently selected from
      (i) halo or R⁵; or
      (ii) C₁₋₄ alkyl, O(C₁₋₄ alkyl), S(C₁₋₄ alkyl), SO(C₁₋₄ alkyl) or SO₂(C₁₋₄ alkyl), each of which is unsubstituted or is substituted with 1, 2 or 3 halo substituents and/or one R⁵ substituent; or
      (iii) two R⁴ groups together with the ring B atoms to which they are joined form a 5- or 6-membered carbocyclic or heterocyclic ring;
      wherein
      - each R⁵ is independently selected from CN, OH, -C(O)NR¹⁰R¹¹, -NR¹⁰R¹¹, -N⁺R¹⁰R¹¹R¹², -NR¹⁰C(NR¹¹)R¹², -C(NR¹⁰)NR¹¹R¹², and -NR¹⁰C(NR¹¹)NR¹²R¹³;
   ∘ *n* is 0, 1, 2, 3 or 4; and
   ∘ each R¹⁰, R¹¹, R¹² and R¹³ is independently H or unsubstituted C₁₋₂ alkyl.
2. A compound according to aspect 1, wherein R¹ is H or unsubstituted C₁ to C₄ alkyl, preferably H; or, where the compound of Formula (I) contains a positively charged nitrogen atom, R¹ maybe absent, such that a COO⁻ group is present and the compound forms a zwitterion.
3. A compound according to aspect 1 or aspect 2, wherein each R² is independently selected from halo, CN and C₁₋₃ alkyl, wherein the C₁₋₃ alkyl group is optionally substituted with 1, 2 or 3 halo substituents; and/or wherein m is 1 or 2.
4. A compound according to any one of the preceding aspects, wherein Z is -NR¹⁰C(O)-, wherein R¹⁰ is H or unsubstituted C₁₋₂ alkyl, preferably R¹⁰ is H.
5. A compound according to any one of the preceding aspects, wherein L is selected from unsubstituted C₁₋₄ alkylene, preferably L is -CH₂-.
6. A compound according to any one of the preceding aspects, wherein Ring B is selected from imidazole, pyrazole, triazole and tetrazole.
7. A compound according to any one of the preceding aspects, wherein
   (i) each R⁴ is independently selected from halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent; wherein each R⁵ is independently selected from -NR¹⁰R¹¹, -N⁺R¹⁰R¹¹R¹², -N^{R10}C(NR¹¹)R¹², -C(NR¹⁰)NR¹¹R¹², and -NR¹⁰C(NR¹¹)NR¹²R¹³; or two R⁴ groups together with the ring B atoms to which they are joined form a 5- or 6-membered heterocyclic ring; and/or
   (ii) n is 1 or 2.
8. A compound according to any one of the preceding aspects, wherein Ring B is substituted with one group R⁴ which is selected from R⁵, unsubstituted O(C₁₋₄ alkyl), unsubstituted S(C₁₋₄ alkyl) and C₁₋₄ alkyl which is unsubstituted or substituted with one R⁵ substituent; wherein R⁵ is independently selected from -NR¹⁰R¹¹, -N⁺R¹⁰R¹¹R¹² and -NR¹⁰C(NR¹¹)NR¹², wherein each R¹⁰, R¹¹, R¹² and R¹³ is independently H or methyl; and Ring B is optionally further substituted with a methyl group; wherein at least one of the substituents on Ring B is on a ring nitrogen atom.
9. A compound according to any one of the preceding aspects, wherein:
   - R¹ is H, or, where the compound of Formula (I) contains a positively charged nitrogen atom, R¹ maybe absent, such that a COO⁻ group is present and the compound forms a zwitterion;
   - each R² is independently selected from F and CN;
   - m is 1 or 2;
   - -Z-L- is -NHC(O)-(CH₂)-;
   - Ring B is selected from imidazole, pyrazole, triazole and tetrazole;
   - *n* is 1 or 2;
   - one group R⁴ is selected from R⁵, unsubstituted O(C₁₋₄ alkyl), unsubstituted S(C₁₋₄ alkyl) and C₁₋₄ alkyl which is unsubstituted or substituted with one R⁵ substituent, wherein R⁵ is independently selected from -NR¹⁰R¹¹, -N⁺R¹⁰R^{N}R¹² and -NR¹⁰C(NR¹¹)NR¹²; and, where present, the other group R⁴ is methyl; and
   - each R¹⁰, R¹¹, R¹² and R¹³ is independently H or methyl.
10. A compound according to any one of the preceding aspects wherein the compound is of Formula (IA) or a pharmaceutically acceptable salt thereof: 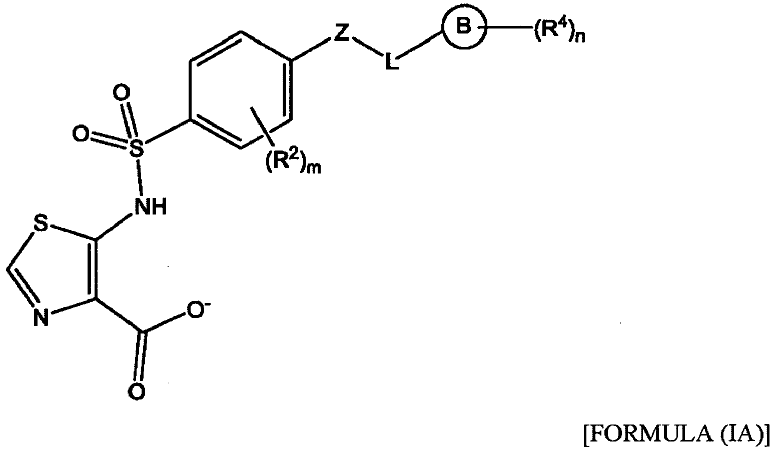
   wherein R², *m,* Z, L and R⁴ are as defined in any one of aspects 1 or 3 to 9;
   Ring B is a five-membered heteroaromatic ring containing 2, 3 or 4 nitrogen atoms, preferably imidazole, pyrazole, triazole or tetrazole, wherein ring B is joined to L via a ring nitrogen atom; and
   *n* is 1, 2, 3 or 4, preferably 1 or 2; and
   wherein at least one R⁴ group is located on a ring nitrogen atom such that the nitrogen atom carries a positive charge.
11. A compound according to aspect 1, which is
   1-[({4-[(4-carboxy-1,3-thiazol-5-yl)sulfamoyl]-2-fluorophenyl}carbamoyl)methyl]-3-methyl-1H-imidazol-3-ium;
   1-[({4-[(4-carboxy-1,3-thiazol-5-yl)sulfamoyl]-2-fluorophenyl}carbamoyl)methyl]-2,3-dimethyl-1H-imidazol-3-ium;
   2-amino-1-[({4-[(4-carboxy-1,3-thiazol-5-yl)sulfamoyl]-2-fluorophenyl}carbamoyl)methyl]-3-methyl-1H-imidazol-3-ium;
   1-[({4-[(4-carboxy-1,3-thiazol-5-yl)sulfamoyl]-2-fluorophenyl}carbamoyl)methyl]-3-methyl-2-(methylsulfanyl)-1H-imidazol-3-ium;
   1-[({4-[(4-carboxy-1,3-thiazol-5-yl)sulfamoyl]-2-fluorophenyl}carbamoyl)methyl]-1H,5H,6H,8H-4*lambda*5-imidazo[2,1-c][1,4]oxazin-4-ylium;
   1-[({4-[(4-carboxy-1,3-thiazol-5-yl)sulfamoyl]-2-fluorophenyl}carbamoyl)methyl]-3,5-dimethyl-1H-imidazol-3-ium;
   1-[({4-[(4-carboxy-1,3-thiazol-5-yl)sulfamoyl]-2-fluorophenyl}carbamoyl)methyl]-3,4-dimethyl-1H-imidazol-3-ium;
   3-amino-1-[({4-[(4-carboxy-1,3-thiazol-5-yl)sulfamoyl]-2-fluorophenyl}carbamoyl)methyl]-2-methyl-1H-pyrazol-2-ium;
   1-[({4-[(4-carboxy-1,3-thiazol-5-yl)sulfamoyl]-2-fluorophenyl}carbamoyl)methyl]-3-methyl-1H-1,2,3-triazol-3-ium;
   1-[({4-[(4-carboxy-1,3-thiazol-5-yl)sulfamoyl]-2-fluorophenyl}carbamoyl)methyl]-2-methyl-1H-pyrazol-2-ium;
   3-(2-aminoethyl)-1-[({4-[(4-carboxy-1,3-1hiazol-5-yl)sulfamoyl]-2-fluorophenyl}carbamoyl)methyl]-1H-imidazol-3-ium;
   3-(2-carbamimidamidoethyl)-1-[({4-[(4-carboxy-1,3-thiazol-5-yl)sulfamoyl]-2-fluorophenyl}carbamoyl)methyl]-1H-imidazol-3-ium;
   1-[({4-[(4-carboxy-1,3-1hiazol-5-yl)sulfamoyl]-2,6-difluorophenyl}carbamoyl)methyl]-3-methyl-1H-imidazol-3-ium;
   3-(2-aminoethyl)-1-[({4-[(4-carboxy-1,3-thiazol-5-yl)sulfamoyl]-2,6-difluorophenyl}carbamoyl)methyl]-1H-imidazol-3-ium;
   1-(2-carbamimidamidoethyl)-3-[({4-[(4-carboxy-1,3-thiazol-5-yl)sulfamoyl]-2,6-difluorophenyl}carbamoyl)methyl]imidazol-1-ium;
   3-(3-aminopropyl)-1-[({4-[(4-carboxy-1,3-thiazol-5-yl)sulfamoyl]-2-fluorophenyl}carbamoyl)methyl]-1H-imidazol-3-ium;
   3-(3-carbamimidamidopropyl)-1-[({4-[(4-carboxy-1,3-thiazol-5-yl)sulfamoyl]-2-fluorophenyl}carbamoyl)methyl]-1H-imidazol-3-ium;
   3-(2-aminoethyl)-1-[({4-[(4-carboxy-1,3-thiazol-5-yl)sulfamoyl]-2-cyanophenyl}carbamoyl)methyl]-1H-imidazol-3-ium;
   3-(2-carbamimidamidoethyl)-1-[({4-[(4-carboxy-1,3-thiazol-5-yl)sulfamoyl]-2-cyanophenyl}carbamoyl)methyl]-1H-imidazol-3-ium;
   3-[({4-[(4-carboxy-1,3-thiazol-5-yl)sulfamoyl]-2-fluorophenyl}carbamoyl)methyl]-1-[2-(dimethylamino)ethyl]imidazol-1-ium;
   3-[({4-[(4-carboxy-1,3-thiazol-5-yl)sulfamoyl]-2-fluorophenyl}carbamoyl)methyl]-1-[2-(trimethylammonio)ethyl]imidazole-1-ium;
   4-[({4-[(4-carboxy-1,3-thiazol-5-yl)sulfamoyl]-2-fluorophenyl}carbamoyl)methyl]-1-methyl-1,2,4-triazol-1-ium;
   1-[({4-[(4-carboxy-1,3-thiazol-5-yl)sulfamoyl]-2,6-difluorophenyl}carbamoyl)methyl]-3-methyl-1H-1,2,3-triazol-3-ium;
   1-[({4-[(4-carboxy-1,3-thiazol-5-yl)sulfamoyl]-2-fluorophenyl}carbamoyl)methyl]-3-ethyl-1H-1,2,3-triazol-3-ium;
   1-[({4-[(4-carboxy-1,3-thiazol-5-yl)sulfamoyl]-2-fluorophenyl}carbamoyl)methyl]-4-methyl-1H-1,2, 4-triazol-4-ium;
   5-amino-1-[({4-[(4-carboxy-1,3-thiazol-5-yl)sulfamoyl]-2-fluorophenyl}carbamoyl)methyl]-4-methyl-1H-1,2,3,4-tetrazol-4-ium;
   or a pharmaceutically acceptable salt thereof.
12. A pharmaceutical composition comprising a compound according to any one of aspects 1 to 11 together with at least one pharmaceutically acceptable carrier or diluent and optionally further comprising (i) an antibiotic agent and/or (ii) a serine-β-lactamase inhibitor.
13. A combination of a compound according to any one of aspects 1 to 11 in combination with one or more of (i) an antibiotic agent and (ii) a serine-β-lactamase inhibitor.
14. A compound according to any one of aspects 1 to 11 for use in the treatment or prevention of bacterial infection by co-administration with an antibiotic agent and/or a serine-β-lactamase inhibitor.
15. An antibiotic agent for use in the treatment or prevention of bacterial infection by co-administration with a compound according to any one of aspects 1 to 11, and optionally a serine-β-lactamase inhibitor.
16. A serine-β-lactamase inhibitor for use in the treatment or prevention of bacterial infection by co-administration with a compound according to any one of aspects 1 to 11, and optionally an antibiotic agent.
17. A composition according to aspect 12, a combination according to aspect 13 or a compound, antibiotic agent or serine-β-lactamase inhibitor for use according to any one of aspects 14 to 16, wherein the antibiotic agent is a β-lactam antibiotic, preferably a β-lactam antibiotic selected from carbapenems, penicillins, cephalosporins and penems.
18. A composition, combination or compound, antibiotic agent or serine-β-lactamase inhibitor for use according to aspect 17, wherein the antibiotic agent is a carbapenem antibiotic, preferably the antibiotic agent is meropenem.
19. A composition, combination or compound, antibiotic agent or serine-β-lactamase inhibitor for use according to any one of aspects 12 to 18 wherein the serine-β-lactamase inhibitor is a compound of Formula (II) or a pharmaceutically acceptable salt thereof, 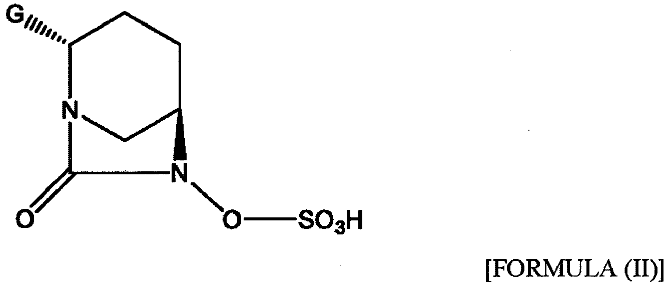 wherein
   ∘ G is selected from -CN and -C(O)NR^{j}R^{k};
   ∘ R^{k} is selected from -W and -Q-W; wherein W is selected from 5- to 6-membered heterocyclyl, R^{j} and -N(R^{j})₂; and Q is selected from -NR^{j}C(O)-, -C(O)-NR^{j}-, C₁₋₃ alkylene, -O-C₁₋₃ alkylene and -N(R^{j})-C₁₋₃ alkylene;
   ∘ each R^{j} is selected from H and unsubstituted C₁₋₃ alkyl, preferably H.
20. A composition, combination or compound, antibiotic agent or serine-β-lactamase inhibitor for use according to any one of aspects 12 to 19, wherein the serine-β-lactamase inhibitor is selected from WCK4234, avibactam, relebactam, zidebactam and nacubactam, or pharmaceutically acceptable salts thereof, wherein preferably the serine-β-lactamase inhibitor is WCK4234 or a pharmaceutically acceptable salt thereof.
21. A compound according to any one of aspects 1 to 11, or a composition or combination according to any one of aspects 12, 13 or 17 to 20 for use in the removal or reduction of antibiotic resistance in Gram-negative bacteria.
22. A compound according to any one of aspects 1 to 11, or a composition or combination according to any one of aspects 12, 13 or 17 to 20 for use in the treatment or prevention of bacterial infection.
23. A compound, antibiotic agent, serine-β-lactamase inhibitor, composition or combination for use according to any one of aspects 14 to 22 wherein the Gram-negative bacteria are selected from Enterobacteriaceae, Pseudomonadaceae and Moraxellaceae, or the bacterial infection is caused by bacteria selected from Enterobacteriaceae, Pseudomonadaceae and Moraxellaceae.
24. A compound, antibiotic agent, serine-β-lactamase inhibitor, composition or combination for use according to aspect 23 wherein the bacteria selected from Enterobacteriaceae, Pseudomonadaceae and Moraxellaceae are selected from *Klebsiella pneumonia, Escherichia coli, Pseudomonas aeruginosa, Burkholderia cepacia* and *Acinetobacter baumannii.*
25. A compound, antibiotic agent, serine-β-lactamase inhibitor, composition or combination for use according to aspect 23 wherein the bacterial infection is caused by Carbapenem Resistant Enterobacteriaceae.

### DETAILED DESCRIPTION OF THE INVENTION

### Definitions

As used herein, a C₁ to C₄ alkyl group is a linear or branched alkyl group containing from 1 to 4 carbon atoms. A C₁ to C₄ alkyl group is often a C₁ to C₃ alkyl group. Examples of C₁ to C₄ alkyl groups include methyl, ethyl, n-propyl, iso-propyl, n-butyl, iso-butyl, sec-butyl, and tert-butyl. A C₁ to C₃ alkyl group is typically a C₁ to C₂ alkyl group. A C₁ to C₂ alkyl group is methyl or ethyl, typically methyl. For the avoidance of doubt, where two alkyl groups are present, the alkyl groups may be the same or different.

As used herein, a C₁ to C₄ alkylene group is an unsubstituted or substituted bidentate moiety obtained by removing two hydrogen atoms from a C₁ to C₄ alkane. The two hydrogen atoms may be removed from the same carbon atom or from different carbon atoms. Typically a C₁ to C₄ alkylene group is a C₁ to C₃ alkylene group. Examples of C₁ to C₄ alkylene groups include methylene, ethylene, n-propylene, iso-propylene, n-butylene, sec-butylene and tert-butylene. A C₁ to C₄ alkylene group is typically a C₁ to C₂ alkylene group. A C₁ to C₂ alkylene group is methylene or ethylene, typically methylene.

As used herein, a C₃ to C₄ alkenylene group is an unsubstituted or substituted bidentate moiety obtained by removing two hydrogen atoms from a C₃ to C₄ alkene. The two hydrogen atoms may be removed from the same carbon atom or from different carbon atoms. Typically a C₃ to C₄ alkenylene group is a C₃ alkenylene group. Examples of C₃ to C₄ alkenylene groups include n-propenylene, iso-propenylene, n-butenylene, sec-butenylene and tert-butenylene. For the avoidance of doubt, where two alkenylene groups are present, the alkenylene groups may be the same or different.

As used herein, a C₃ to C₄ alkynylene group is an unsubstituted or substituted bidentate moiety obtained by removing two hydrogen atoms from a C₃ to C₄ alkyne. The two hydrogen atoms may be removed from the same carbon atom or from different carbon atoms. Typically a C₃ to C₄ alkynylene group is a C₃ alkynylene group. Examples of C₃ to C₄ alkynylene groups include n-propynylene, iso-propynylene, n-butynylene, sec-butynylene and tert-butynylene.

An alkyl, alkylene, alkenylene or alkynylene group as used herein may be unsubstituted or substituted. Unless otherwise stated, substituted alkyl, alkylene, alkenylene and alkynylene groups typically carry one or more, e.g. 1, 2, 3 or 4, such as one, two or three e.g. one, or two, e.g. one substituent selected from halogen, -CN, -OR¹⁰, -C(O)NR¹⁰R¹¹, -NR¹⁰R¹¹, -N⁺R¹⁰R¹¹R¹², -NR¹⁰C(NR¹¹)R¹², -C(NR¹⁰)NR¹¹R¹² and -NR¹⁰C(NR¹¹)NR¹²R¹³, wherein R¹⁰, R¹¹, R¹² and R¹³ are as defined herein. Preferred substituents on an alkyl, alkylene, alkenylene or alkynylene group are halogen, -CN, -OR¹⁰, -NR¹⁰R¹¹ and -N⁺R¹⁰R¹¹R¹², in particular halogen, -CN, -OR¹⁰ and -NR¹⁰R¹¹, wherein R¹⁰, R¹¹ and R¹² are as defined herein. The substituents on a substituted alkyl, alkenyl or alkynyl group are typically themselves unsubstituted unless otherwise stated. Where more than one substituent is present, these may be the same or different.

As used herein, a halogen or halo group is typically chlorine, fluorine, bromine or iodine and is preferably chlorine, bromine or fluorine, especially chorine or fluorine, especially fluorine.

A 5- to 6- membered carbocyclic group is a cyclic hydrocarbon containing 5 or 6 carbon atoms. A carbocyclic group may be saturated or partially unsaturated, but is typically partially unsaturated. A 5- to 6- membered partially unsaturated carbocyclic group is a cyclic hydrocarbon containing 5 or 6 carbon atoms and containing 1 or 2, e.g. 1 double bond. A carbocyclic group may be a fused to a further ring, e.g. it may be fused to Ring B, as defined herein. Examples of 5- to 6- membered carbocyclic groups include the saturated groups cyclopentyl and cyclohexyl, and the partially unsaturated groups cyclopentenyl and cyclohexenyl.

A 5- to 6- membered heterocyclic group is a cyclic group containing 5 or 6 atoms selected from C, O, N and S in the ring, including at least one heteroatom, and typically one or two heteroatoms. The heteroatom or heteroatoms are typically selected from O, N, and S. For example, where the heterocyclic group may be a nitrogen-containing heterocyclic group containing one nitrogen atom and optionally a further heteroatom selected from O, N and S, in particular O. A heterocyclic group may be saturated or partially unsaturated. A 5- to 6-membered partially unsaturated heterocyclic group is a cyclic group containing 5 or 6 atoms selected from C, O, N and S in the ring and containing 1 or 2, e.g. 1 double bond.

Examples of 5- and 6- membered saturated heterocyclic groups include piperazine, piperidine, morpholine, 1,3-oxazinane, pyrrolidine, imidazolidine, and oxazolidine, including quaternised derivatives thereof. Examples of 5- and 6- membered partially saturated heterocyclic groups include tetrahydropyrazine, tetrahydropyridine, dihydro-1,4-oxazine, tetrahydropyrimidine, dihydro-1,3-oxazine, dihydropyrrole, dihydroimidazole and dihydrooxazole, including quaternised derivatives thereof. Dihydro-1,4-oxazine, dihydro-1,3-oxazine and dihydrooxazole, including quaternised derivatives thereof, are preferred.

For the avoidance of doubt, a heterocyclic group may be fused to a further ring system, e.g. it may be fused to Ring B. Where a heterocyclic group is fused to Ring B and includes a Ring B nitrogen atom, the nitrogen atom may be quaternised.

As used herein, a 5-membered heteroaromatic group is a substituted or unsubstituted monocyclic aromatic group containing 5 atoms in the ring portion, including at least one heteroatom, for example 1, 2, 3 or 4 heteroatoms, typically selected from O, S and N.

A carbocyclic, heterocyclic, phenyl or heteroaromatic group may be unsubstituted or substituted as described herein. For example, a carbocyclic, heterocyclic, phenyl or heteroaromatic group may be unsubstituted or substituted with 1, 2 or 3, typically 1 or 2 such as e.g. 1 substituent. Suitable substituents include those defined as R² and R⁴ herein. The substituents on a substituted carbocyclic, heterocyclic, phenyl or heteroaromatic group are typically themselves unsubstituted, unless otherwise stated. Substituents may be present on one or more of the carbon and, where present, nitrogen atoms in a ring.

Compounds of the invention may comprise heterocyclic or heteroaromatic groups comprising at least one nitrogen atom. In such compounds, said nitrogen atom(s) are independently selected from secondary, tertiary and quaternary nitrogen atom(s). A quaternary nitrogen atom is present when the compound comprises a quaternised derivative of one or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p*-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts.

Where the compound of Formula (I) contains a positively charged nitrogen atom, the compound may exist as a zwitterion, where R¹ is absent, thus leaving a COO⁻ group. Such compounds may also be provided in the form of a pharmaceutically acceptable salt. Suitable salts include those formed with pharmaceutically acceptable acids, which provide a proton to the COO⁻ group, and a counter-ion to balance the positive charge on the quaternary nitrogen atom. Suitable pharmaceutically acceptable acids include hydrochloric acid, sulphonic acids including methanesulphonic acid and toluene sulphonic acid, ascorbic acid and citric acid. Hydrochloric acid and sulphonic acids are preferred, in particular hydrochloric acid. Alternatively, zwitterions can be combined with pharmaceutically acceptable bases as mentioned above, for example, alkali metal (e.g. sodium or potassium) and alkali earth metal (e.g. calcium or magnesium) hydroxides.

In Formula (I), the stereochemistry is not limited. In particular, compounds of Formula (I) containing one or more chiral centre may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound according to Formula (I) which is enantiomerically or diasteriomerically pure. Typically, a compound of the invention comprises by weight at least 60%, such as at least 75%, 90%, or 95% of a single enantiomer or diastereomer. Preferably, the compound is substantially optically pure.

### Compounds of the Invention

Typically, in Formula (I), R¹ is selected from H and R^{1a}, typically from H and unsubstituted C₁ to C₄ alkyl. More preferably, R¹ is H. R^{1a} is typically an unsubstituted C₁ to C₄ alkyl group, such as an unsubstituted C₁ to C₂ alkyl group. More preferably, R^{1a} is methyl or t-butyl. In one preferred embodiment, the compound of Formula (I) contains a quaternary nitrogen atom carrying a positive charge, for example where a nitrogen atom in Ring B is substituted with an R⁴ group. In this case, R¹ is preferably absent such that a COO⁻ group is present and the compound forms a zwitterion. Alternatively, in this embodiment, R¹ may be present and is as defined above, and the compound is in the form of a pharmaceutically acceptable salt where a counter-ion (e.g. chloride) is present to balance the positive charge on the quaternary nitrogen atom.

Typically, in Formula (I), each R² is independently selected from halo, CN and C₁₋₃ alkyl, wherein the C₁₋₃ alkyl group is optionally substituted with 1, 2 or 3 halogen substituents. More preferably each R² is independently selected from halo and CN. Fluorine is a preferred halogen group. Typically, m is 0, 1 or 2, preferably 1 or 2. Most preferably, the phenyl ring carries either one R² substituent which is selected from CN and F, or two R² substituents, each of which is F. The substituents, when present, are preferably located *ortho* to the group -Z-L-.

Typically, in Formula (I), Z is selected from -NR¹⁰C(O)-, -C(O)NR¹⁰-, and -NR¹⁰C(O)NR¹¹-. Preferably, Z is -NR¹⁰C(O)-, wherein R¹⁰ is H or unsubstituted C₁₋₂ alkyl, preferably H or methyl. Most preferably Z is -NHC(O)-.

In Formula (I), L is unsubstituted or is substituted with 1 or 2 substituents selected from halogen, -OR¹⁰, -NR¹⁰R¹¹ and -N⁺R¹⁰R¹¹R¹². Preferred substituents are halogen, -OR¹⁰ and -NR¹⁰R¹¹. Typically, any halogen substituent on L is not located α to -Z- or α to Ring B. Typically, any -OR¹⁰, -NR¹⁰R¹¹ or -N⁺R¹⁰R¹¹R¹² substituent on L is not located α to Ring B.

Typically, in Formula (I), L is unsubstituted or is substituted with 1 substituent selected from halogen, -OR¹⁰, and -NR¹⁰R¹¹; preferably L is unsubstituted.

L is typically selected from C₁₋₄ alkylene, e.g. C₁₋₃ alkylene, preferably unsubstituted C₁₋₄ alkylene or unsubstituted C₁₋₃ alkylene. Most preferably L is -CH₂-.

Typically, therefore, in Formula (I):
- Z is selected from -NR¹⁰C(O)-, -C(O)NR¹⁰-, and -NR¹⁰C(O)NR¹¹-; and
- L is unsubstituted C₁₋₄ alkylene.

Preferably, -Z-L- is -NR¹⁰C(O)-(CH₂)₁₋₄-, wherein R¹⁰ is H or methyl; more preferably -Z-L- is -NHC(O)-(CH₂)₁₋₃-; most preferably -Z-L- is -NHC(O)-(CH₂)-.

In Formula (I), Ring B is a five-membered heteroaromatic ring containing 1, 2, 3 or 4 nitrogen atoms, wherein ring B is joined to L via a ring nitrogen atom. Ring B may contain one or more further heteroatoms, e.g. one or more O or S atoms. Preferably, Ring B contains from one to four nitrogen atoms, most preferably two to four nitrogen atoms, and the remaining ring atoms are carbon atoms. Preferably, Ring B is selected from imidazole, pyrazole, triazole and tetrazole.

Ring B maybe unsubstituted or it may carry from one to four substituents R⁴, i.e. *n* is 0, 1, 2, 3 or 4. Preferably, *n* is 0, 1 or 2, most preferably 1 or 2. Where *n* is 2, most preferably at least one of the substituents R⁴ is methyl or ethyl, most preferably methyl. The substituents on B may be on any ring atom, including any ring carbon atom or any ring nitrogen atom. For the avoidance of doubt, where a substituent is present on a ring nitrogen atom, the nitrogen atom will carry a positive charge. Preferred compounds of Formula (I) are those wherein at least one ring nitrogen atom is substituted. Such compounds typically exist in the form of a zwitterion, where R¹ is absent to provide a COO⁻ group on the thiazole ring.

In Formula (I), R⁴ is selected from:
(i) halo or R⁵; or
(ii) C₁₋₄ alkyl, O(C₁₋₄ alkyl), S(C₁₋₄ alkyl), SO(C₁₋₄ alkyl) or SO₂(C₁₋₄ alkyl), any of which may optionally be substituted with 1, 2 or 3 halo substituents and/or one R⁵ substituent, or
(iii) two R⁴ groups together with the ring B atoms to which they are joined form a 5- or 6-membered carbocyclic or heterocyclic ring.

Preferably, R⁴ is selected from (i) or (ii) above.

When R⁴ is selected from (i) or (ii) above, then each group R⁴ is typically independently selected from halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent. Preferably, when R⁴ is selected from (i) or (ii), each group R⁴ is independently selected from R⁵, unsubstituted O(C₁₋₄ alkyl), unsubstituted S(C₁₋₄ alkyl) and C₁₋₄ alkyl which is unsubstituted or substituted with one R⁵ substituent.

Each R⁵ is typically independently selected from -NR¹⁰R¹¹, -N⁺R¹⁰R¹¹R¹², -NR¹⁰C(NR¹¹)R¹², -C(NR¹⁰)NR¹¹R¹² and -NR¹⁰C(NR¹¹)NR¹²R¹³, preferably from -NR¹⁰R¹¹, -N⁺R¹⁰R¹¹R¹² and -NR¹⁰C(NR¹¹)NR¹²R¹³. In this aspect, each R¹⁰, R¹¹, R¹² and R¹³ is independently H or unsubstituted C₁₋₂ alkyl, preferably each R¹⁰, R¹¹, R¹² and R¹³ is independently H or methyl. Thus, preferred groups R⁵ are -NR¹⁰R¹¹, -N⁺R¹⁰R¹¹R¹², -NR¹⁰C(NR¹¹)R¹², -C(NR¹⁰)NR¹¹R¹² and -NR¹⁰C(NR¹¹)NR¹²R¹³, in particular -NR¹⁰R¹¹, -N⁺R¹⁰R¹¹R¹² and -NR¹⁰C(NR¹¹)NR¹²R¹³, wherein each R¹⁰, R¹¹, R¹² and R¹³ is independently H or methyl. Most preferred groups R⁵ are -NH₂, -NMe₂, -N⁺Me₃ and -NHC(NH)NH₂.

Where two R⁴ groups together with the ring B atoms to which they are joined form a 5- or 6-membered carbocyclic or heterocyclic ring, typically the ring is a heterocyclic ring, preferably a heterocyclic ring containing at least one heteroatom selected from nitrogen, oxygen and sulphur. The ring may contain one or more heteroatoms in the portion which is fused to Ring B. The ring may contain one or more heteroatoms in the unfused portion of the ring. For instance, the ring may contain none or one nitrogen atom in the portion which is fused to Ring B, and none or one oxygen atom in the unfused portion. Preferred rings contain one nitrogen and one oxygen atom, with the remainder typically being carbon atoms.

The ring formed by two R⁴ groups is typically a partially unsaturated ring, in particular, the two groups R⁴ may join to form a ring which is unsaturated in the portion which is fused to ring B, but which is saturated in the remainder of the ring. Preferred rings include dihydrooxazine and dihydrooxazole, in particular, dihydrooxazine.

The ring formed by two R⁴ groups is itself unsubstituted.

Therefore, in Formula (i), typically each R⁴ is independently selected from
- halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent, wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹², and -NR¹⁰C(NR¹¹)NR¹²R¹³; or
- two R⁴ groups together with the Ring B atoms to which they are joined form a 5- or 6-membered heterocyclic ring.

Preferably, each R⁴ is independently selected from
- R⁵, unsubstituted O(C₁₋₄ alkyl), unsubstituted S(C₁₋₄ alkyl) and C₁₋₄ alkyl which is unsubstituted or substituted with one R⁵ substituent, wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹², and -NR¹⁰C(NR¹¹)NR¹²R¹³; or
- two R⁴ groups together with the Ring B atoms to which they are joined form a 5- or 6-membered heterocyclic ring.

More preferably, each R⁴ is independently selected from
- R⁵, unsubstituted O(C₁₋₄ alkyl), unsubstituted S(C₁₋₄ alkyl) and C₁₋₄ alkyl which is unsubstituted or substituted with one R⁵ substituent, wherein each R⁵ is independently selected from -NR¹⁰R¹¹, -N⁺R¹⁰R¹¹R¹² and -NR¹⁰C(NR¹¹)NR¹²R¹³, wherein each R¹⁰, R¹¹, R¹² and R¹³ is independently H or methyl; or
- two R⁴ groups together with the Ring B atoms to which they are joined form a 5- or 6-membered heterocyclic ring containing one nitrogen and one oxygen atom.

Typically, at least one substituent is present on a ring nitrogen atom of Ring B. Further, typically where two or more R⁴ groups are present on Ring B, one R⁴ is selected from the R⁴ groups as defined above, whilst the remainder are methyl groups. Preferably, up to two R⁴ groups are present, one group being selected from the R⁴ groups as defined above and the other, if present, being methyl.

Therefore, in a preferred embodiment, Ring B is substituted with one group R⁴ which is selected from R⁵, unsubstituted O(C₁₋₄ alkyl), unsubstituted S(C₁₋₄ alkyl) and C₁₋₄ alkyl which is unsubstituted or substituted with one R⁵ substituent; wherein R⁵ is selected from -NR¹⁰R¹¹, -N⁺R¹⁰R¹¹R¹², -NR¹⁰C(NR¹¹)R¹², -C(NR¹⁰)NR¹¹R¹² and -NR¹⁰C(NR¹¹)NR¹²R¹³; and Ring B is optionally further substituted with a methyl group; or
two R⁴ groups together with the ring B atoms to which they are joined form a 5- or 6-membered heterocyclic ring.

More preferably, Ring B is substituted with one group R⁴ which is selected from R⁵, unsubstituted O(C₁₋₄ alkyl), unsubstituted S(C₁₋₄ alkyl) and C₁₋₄ alkyl which is unsubstituted or substituted with one R⁵ substituent; wherein R⁵ is independently selected from -NR¹⁰R¹¹,-N⁺R¹⁰R¹¹R¹² and -NR¹⁰C(NR¹¹)NR¹², wherein each R¹⁰, R¹¹, R¹² and R¹³ is independently H or methyl; and Ring B is optionally further substituted with a methyl group;
preferably wherein at least one of the substituents on Ring B is on a ring nitrogen atom.

R¹⁰, R¹¹, R¹² and R¹³ are independently H or unsubstituted C₁₋₂ alkyl, preferably H or methyl, for example H.

Preferred compounds of Formula (I) are those wherein:
- R¹ is selected from H and unsubstituted C₁ to C₄ alkyl, or, where the compound of Formula (I) contains a positively charged nitrogen atom, R¹ may be absent, such that a COO⁻ group is present and the compound forms a zwitterion;
- each R² is independently selected from halo, CN and C₁₋₃ alkyl, wherein the C₁₋₃ alkyl group is optionally substituted with 1, 2 or 3 halogen substituents;
- *m* is 0, 1 or 2;
- Z is selected from -NR¹⁰C(O)-, -C(O)NR¹⁰-, and -NR¹⁰C(O)NR¹¹-;
- L is unsubstituted C₁₋₄ alkylene;
- Ring B is a five-membered heteroaromatic ring containing 1, 2, 3 or 4 nitrogen atoms, wherein ring B is joined to L via a ring nitrogen atom;
- *n* is 0, 1 or 2;
- each R⁴ is independently selected from:
   - halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent, wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹² and -NR¹⁰C(NR¹¹)NR¹²R¹³; or
   - two R⁴ groups together with the Ring B atoms to which they are joined form a 5- or 6-membered heterocyclic ring; and
- each R¹⁰, R¹¹, R¹² and R¹³ is independently H or unsubstituted C₁₋₂ alkyl.

More preferred compounds of Formula (I) are those wherein:
- R¹ is H, or, where the compound of Formula (I) contains a positively charged nitrogen atom, R¹ may be absent, such that a COO⁻ group is present and the compound forms a zwitterion;
- each R² is independently selected from halo and CN;
- *m* is 1 or 2;
- Z is -NR¹⁰C(O)-;
- L is unsubstituted C₁₋₃ alkylene;
- Ring B is selected from imidazole, pyrazole, triazole and tetrazole;
- *n* is 1 or 2;
- each R⁴ is independently selected from
   - R⁵, unsubstituted O(C₁₋₄ alkyl), unsubstituted S(C₁₋₄ alkyl) and C₁₋₄ alkyl which is unsubstituted or substituted with one R⁵ substituent, wherein each R⁵ is independently selected from -NR¹⁰R¹¹, -N⁺R¹⁰R¹¹R¹², -NR¹⁰C(NR¹¹)R¹², ⁻C(NR¹⁰)NR¹¹R¹², and -NR¹⁰C(NR¹¹)NR¹²R¹³; or
   - two R⁴ groups together with the Ring B atoms to which they are joined form a 5- or 6-membered heterocyclic ring; and
- each R¹⁰, R¹¹, R¹² and R¹³ is independently H or methyl.

Particularly preferred compounds of Formula (I) are those wherein:
- R¹ is H, or, where the compound of Formula (I) contains a positively charged nitrogen atom, R¹ may be absent, such that a COO⁻ group is present and the compound forms a zwitterion;
- each R² is independently selected from F and CN;
- *m* is 1 or 2;
- -Z-L- is -NHC(O)-(CH₂)-;
- Ring B is selected from imidazole, pyrazole, triazole and tetrazole;
- *n* is 1 or 2;
- one group R⁴ is selected from R⁵, unsubstituted O(C₁₋₄ alkyl), unsubstituted S(C₁₋₄ alkyl) and C₁₋₄ alkyl which is unsubstituted or substituted with one R⁵ substituent, wherein R⁵ is independently selected from -NR¹⁰R¹¹, -N⁺R¹⁰R¹¹R¹² and -NR¹⁰C(NR¹¹)NR¹²; and, where present, the other group R⁴ is methyl; and
- each R¹⁰, R¹¹, R¹² and R¹³ is independently H or methyl.

In one embodiment, the compound as defined above is in the form of a zwitterion of Formula (IA), or a pharmaceutically acceptable salt thereof: 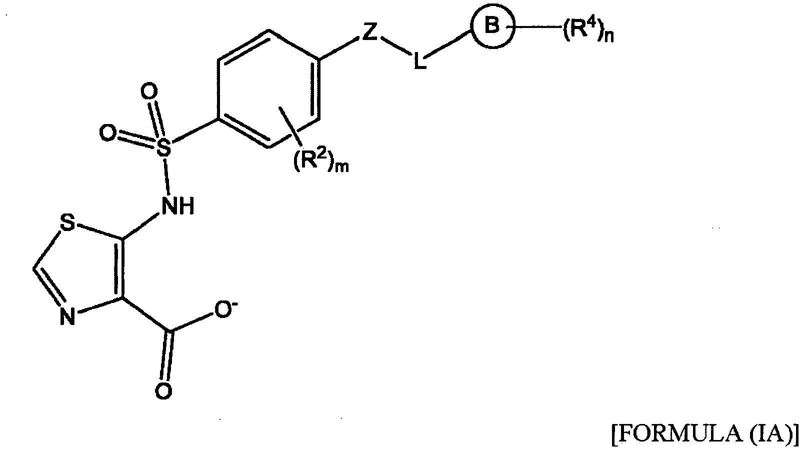 wherein R², *m*, Z, L and R⁴ are as defined above, and wherein Ring B is a five-membered heteroaromatic ring containing 2, 3 or 4 nitrogen atoms, wherein ring B is joined to L via a ring nitrogen atom. Ring B may contain one or more further heteroatoms, e.g. one or more O or S atoms. Preferably, Ring B is selected from imidazole, pyrazole, triazole and tetrazole.

In this aspect, Ring B carries from one to four substituents R⁴, i.e. *n* is 1, 2, 3 or 4, preferably 1 or 2, and at least one substituent is on a ring nitrogen atom such that the nitrogen atom carries a positive charge.

Preferred compounds of Formula (IA) are those wherein:
- each R² is independently selected from halo, CN and C₁₋₃ alkyl, wherein the C₁₋₃ alkyl group is optionally substituted with 1, 2 or 3 halogen substituents;
- *m* is 0, 1 or 2;
- Z is selected from -NR¹⁰C(O)-, -C(O)NR¹⁰-, and -NR¹⁰C(O)NR¹¹-;
- L is unsubstituted C₁₋₄ alkylene;
- Ring B is a five-membered heteroaromatic ring containing 2, 3 or 4 nitrogen atoms, wherein ring B is joined to L via a ring nitrogen atom;
- *n* is 1 or 2;
- each R⁴ is independently selected from:
   - halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent, wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹² and-NR¹⁰C(NR¹¹)NR¹²R¹³; or
   - two R⁴ groups together with the Ring B atoms to which they are joined form a 5- or 6-membered heterocyclic ring;
   - wherein at least one substituent R⁴ is on a nitrogen atom of Ring B; and
- each R¹⁰, R¹¹, R¹² and R¹³ is independently H or unsubstituted C₁₋₂ alkyl.

More preferred compounds of Formula (IA) are those wherein:
- each R² is independently selected from halo and CN;
- *m* is 1 or 2;
- Z is -NR¹⁰C(O)-;
- L is unsubstituted C₁₋₃ alkylene;
- Ring B is selected from imidazole, pyrazole, triazole and tetrazole;
- *n* is 1 or 2;
- each R⁴ is independently selected from
   - R⁵, unsubstituted O(C₁₋₄ alkyl), unsubstituted S(C₁₋₄ alkyl) and C₁₋₄ alkyl which is unsubstituted or substituted with one R⁵ substituent, wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹², and -NR¹⁰C(NR¹¹)NR¹²R¹³; or
   - two R⁴ groups together with the Ring B atoms to which they are joined form a 5- or 6-membered heterocyclic ring;
   - wherein at least one substituent R⁴ is on a nitrogen atom of Ring B; and
- each R¹⁰, R¹¹, R¹² and R¹³ is independently H or methyl.

Particularly preferred compounds of Formula (IA) are those wherein:
- each R² is independently selected from F and CN;
- *m* is 1 or 2;
- -Z-L- is -NHC(O)-(CH₂)-;
- Ring B is selected from imidazole, pyrazole, triazole and tetrazole;
- *n* is 1 or 2;
- one group R⁴ is selected from R⁵, unsubstituted O(C₁₋₄ alkyl), unsubstituted S(C₁₋₄ alkyl) and C₁₋₄ alkyl which is unsubstituted or substituted with one R⁵ substituent, wherein R⁵ is independently selected from -NR¹⁰R¹¹, -N⁺R¹⁰R¹¹R¹² and -NR¹⁰C(NR¹¹)NR¹²; and, where present, the other group R⁴ is methyl; wherein at least one substituent R⁴ is on a nitrogen atom of Ring B; and
- each R¹⁰, R¹¹, R¹² and R¹³ is independently H or methyl.

Particularly preferred compounds of the invention are:
1. 1-[({4-[(4-carboxy-1,3-thiazol-5-yl)sulfamoyl]-2-fluorophenyl}carbamoyl)methyl]-3-methyl-1H-imidazol-3-ium;
2. 1-[({4-[(4-carboxy-1,3-thiazol-5-yl)sulfamoyl]-2-fluorophenyl}carbamoyl)methyl]-2,3-dimethyl-1H-imidazol-3-ium;
3. 2-amino-1-[({4-[(4-carboxy-1,3 -thiazol-5-yl)sulfamoyl]-2-fluorophenyl}carbamoyl)methyl]-3-methyl-1H-imidazol-3-ium;
4. 1-[({4-[(4-carboxy-1,3-thiazol-5-yl)sulfamoyl]-2-fluorophenyl}carbamoyl)methyl]-3-methyl-2-(methylsulfanyl)-1H-imidazol-3-ium;
5. 1-[({4-[(4-carboxy-1,3-thiazol-5-yl)sulfamoyl]-2-fluorophenyl}carbamoyl)methyl]-1H,5H,6H,8H-4*lambda*5-imidazo[2,1-c][1,4]oxazin-4-ylium;
6. 1-[({4-[(4-carboxy-1,3-thiazol-5-yl)sulfamoyl]-2-fluorophenyl}carbamoyl)methyl]-3,5-dimethyl-1H-imidazol-3-ium;
7. 1-[({4-[(4-carboxy-1,3-thiazol-5-yl)sulfamoyl]-2-fluorophenyl}carbamoyl)methyl]-3,4-dimethyl-1H-imidazol-3-ium;
8. 3-amino-1-[({4-[(4-carboxy-1,3-thiazol-5-yl)sulfamoyl]-2-fluorophenyl}carbamoyl)methyl]-2-methyl-1H-pyrazol-2-ium;
9. 1-[({4-[(4-carboxy-1,3-thiazol-5-yl)sulfamoyl]-2-fluorophenyl}carbamoyl)methyl]-3-methyl-1H-1,2,3-triazol-3-ium;
10. 1-[({4-[(4-carboxy-1,3-thiazol-5-yl)sulfamoyl]-2-fluorophenyl}carbamoyl)methyl]-2-methyl-1H-pyrazol-2-ium;
11. 3-(2-aminoethyl)-1-[({4-[(4-carboxy-1,3-thiazol-5-yl)sulfamoyl]-2-fluorophenyl}carbamoyl)methyl]-1H-imidazol-3-ium;
12. 3-(2-carbamimidamidoethyl)-1-[({4-[(4-carboxy-1,3-thiazol-5-yl)sulfamoyl]-2-fluorophenyl}carbamoyl)methyl]-1H-imidazol-3-ium;
13. 1-[({4-[(4-carboxy-1,3-thiazol-5-yl)sulfamoyl]-2,6-difluorophenyl}carbamoyl)methyl]-3-methyl-1H-imidazol-3-ium;
14. 3-(2-aminoethyl)-1-[({4-[(4-carboxy-1,3-thiazol-5-yl)sulfamoyl]-2,6-difluorophenyl}carbamoyl)methyl]-1H-imidazol-3-ium;
15. 1-(2-carbamimidamidoethyl)-3-[({4-[(4-carboxy-1,3-thiazol-5-yl)sulfamoyl]-2,6-difluorophenyl}carbamoyl)methyl]imidazol-1-ium;
16. 3-(3-aminopropyl)-1-[({4-[(4-carboxy-1,3-thiazol-5-yl)sulfamoyl]-2-fluorophenyl}carbamoyl)methyl]-1H-imidazol-3-ium;
17. 3-(3-carbamimidamidopropyl)-1-[({4-[(4-carboxy-1,3-thiazol-5-yl)sulfamoyl]-2-fluorophenyl}carbamoyl)methyl]-1H-imidazol-3-ium;
18. 3-(2-aminoethyl)-1-[({4-[(4-carboxy-1,3-thiazol-5-yl)sulfamoyl]-2-cyanophenyl}carbamoyl)methyl]-1H-imidazol-3-ium;
19. 3-(2-carbamimidamidoethyl)-1-[({4-[(4-carboxy-1,3-thiazol-5-yl)sulfamoyl]-2-cyanophenyl}carbamoyl)methyl]-1H-imidazol-3-ium;
20. 3-[({4-[(4-carboxy-1,3-thiazol-5-yl)sulfamoyl]-2-fluorophenyl}carbamoyl)methyl]-1-[2-(dimethylamino)ethyl]imidazol-1-ium;
21. 3-[({4-[(4-carboxy-1,3-thiazol-5-yl)suliamoyl]-2-fluorophenyl}carbamoyl)methyl]-1-[2-(trimethylammonio)ethyl]imidazole-1-ium;
22. 4-[({4-[(4-carboxy-1,3-thiazol-5-yl)sulfamoyl]-2-fluorophenyl}carbamoyl)methyl]-1-methyl-1,2,4-triazol-1-ium;
23. 1-[({4-[(4-carboxy-1,3-thiazol-5-yl)sulfamoyl]-2,6-difluorophenyl}carbamoyl)methyl]-3-methyl-1H-1,2,3-triazol-3-ium;
24. 1-[({4-[(4-carboxy-1,3-thiazol-5-yl)sulfamoyl]-2-fluorophenyl}carbamoyl)methyl]-3-ethyl-1H-1,2,3-triazol-3-ium;
25. 1-[({4-[(4-carboxy-1,3-thiazol-5-yl)sulfamoyl]-2-fluorophenyl}carbamoyl)methyl]-4-methyl-1H-1,2,4-triazol-4-ium;
26. 5-amino-1-[({4-[(4-carboxy-1,3-thiazol-5-yl)sulfamoyl]-2-fluorophenyl}carbamoyl)methyl]-4-methyl-1H-1,2,3,4-tetrazol-4-ium;
and pharmaceutically acceptable salts of each of these compounds.

### Synthesis

The compounds of the invention can be prepared by any suitable method. Detailed general synthetic routes for representative compounds of the invention are set out below and in the Examples.

In summary, compounds of the invention can typically be prepared in a reaction according to the following scheme:

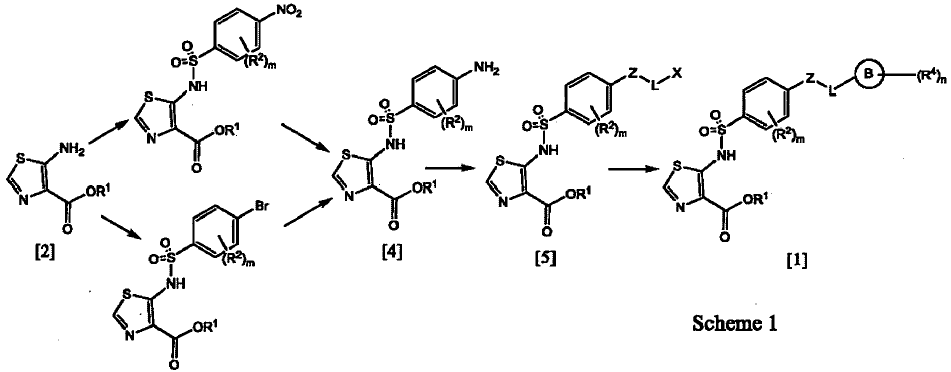

Starting material [2] is readily available or can be obtained by routine methods known to the skilled person in the art. Anilines [4] can be obtained via a number of different routes. For example, reaction of aminothiazole [2] with an arylsulphonyl chloride containing a nitro group, followed by standard reduction of nitro to amino, provides aniline [4]. The reaction may proceed directly from the aminothiazole [2], or from a compound where a protecting group such as p-methoxybenzyl is present on the amine group. Alternatively, [2] may be reacted with an arylsulphonyl chloride containing, for example, a bromine atom on the aryl ring, followed by standard Buchwald animation, to access the same aniline [4]. Suitable nucleophiles for the Buchwald amination include ammonia and benzophenone imine followed by hydrolysis.

Aniline [4] can be reacted with a chloroacetyl chloride to give key intermediate chloroacetamide [5], where -Z-L- is -NHCO-CH₂- and X is chlorine. [5] can be reacted with azoles or substituted azoles to provide compounds of the invention [1]. Alkylation with an appropriate alkylhalide may be used to generate quaternised heterocycles. Suitable protection of reactive groups may be used during the synthesis. For example, R1 may be protected as a t-butyl group prior to reaction and deprotected with trifluoroacetic acid (TFA) as a final step.

Alternative routes to the compounds of the invention, including those where -Z-L- is other than -NHCO-CH₂-, include, for example, that set out in Scheme 2 below:

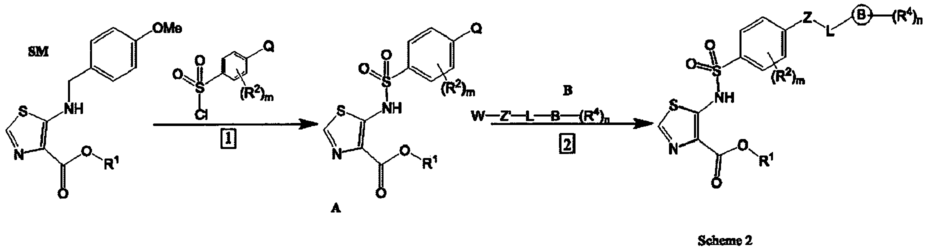

Starting material SM is readily available and can, for example, be prepared using the methods described in WO 2014/198849. The disclosure of WO 2014/198849 regarding the formation of compound SM and its analogues is incorporated by reference. Reaction of SM with a sulphonyl chloride (reaction step 1) yields a thiazole sulphonamide derivative (**A**). Reaction of **A** with W-Z'-L-Ring B-(R⁴)ₙ yields compounds [1]. In the above scheme, Q and W are complementary reactive groups which react together to couple **A** to W-Z-L-Ring B. For example, Q can be bromine and -Z-W can be -C(O)NH₂ so that Q and W react together via Buchwald chemistry. Alternatively, Q can be -NH₂ and -Z-W can be -C(O)OH so that Q and W react together in a standard peptide coupling reaction using reagents such as HATU. Other methods of coupling compounds are well known to those skilled in the art. Suitable protection strategies may be used in order to protect the acid at R¹ and any reactive groups on Ring B, as needed. Detailed synthetic routes to exemplary compounds of the invention are set out below.

### Compositions and Combination Therapies

The compounds of the invention may be provided in a pharmaceutical composition, the pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

As explained above, the compounds of the invention are useful in treating or preventing bacterial infection. In particular, they are inhibitors of metallo-β-lactamase (MBL) enzymes and are therefore useful for removing or reducing resistance of Gram-negative bacteria to antibiotics. The compounds may be used alone or they may be used in combination therapies with antibiotic agents, to enhance the action of the antibiotic agent. The compounds may also be used in combination with serine-β-lactamase (SBL) inhibitors, in particular where the bacteria causing the infection are resistant to treatment by an antibiotic agent alone and the resistance is, or is suspected of being, caused at least in part by serine-β-lactamase enzymes.

The present invention therefore also provides a combination of a compound as described herein and (i) an antibiotic agent. Combinations comprising an antibiotic agent are referred to herein as antibiotic combinations.

The present invention also provides combinations of the compounds of the invention with a serine-β-lactamase (SBL) inhibitor. Thus, the invention also provides a combination of (i) a compound of the invention; and one or more of (ii) a serine-β-lactamase (SBL) inhibitor; and (iii) an antibiotic agent. Combinations with an SBL inhibitor are referred to herein as SBLi combinations. Combinations of all three active agents (i) to (iii) are referred to herein as "triple combinations". The triple combinations are combinations of the above three active agents (i) to (iii) but may also comprise further active agents if desired.

In the combinations of the invention, the active agents may each be provided in a single formulation or one or more of them may be separately formulated. Where separately formulated, the two or more agents may be administered simultaneously or separately. Active agents which are separately formulated may be provided in the form of a kit, optionally together with instructions for their administration. For example, the kit may comprise (i) a composition comprising a compound of the invention; and one or both of (ii) a composition comprising an antibiotic agent and (iii) a composition comprising an SBL inhibitor, optionally together with instructions for their use. Where two or more active agents are formulated together, the two or more active agents may be provided as a pharmaceutical composition. The pharmaceutical composition may therefore comprise (i) a compound of the invention as described herein; (ii) a pharmaceutically acceptable carrier or diluent; and one or both of (iii) an antibiotic agent, and (iv) a serine-β-lactamase (SBL) inhibitor.

Thus, in the antibiotic combinations, the compound of the invention and the antibiotic agent may be formulated together or separately. In the SBLi combinations, the compound of the invention and the SBLi inhibitor may be formulated together or separately. In the triple combinations, the compound of the invention, the SBL inhibitor and the antibiotic agent may each be provided in a single formulation, or they may be separately formulated. Alternatively, two of the components may be provided in a single formulation and the remaining component may be provided separately. In other words, the compound of the invention may be formulated with the SBL inhibitor and the antibiotic agent, or the compound of the invention may be formulated with the SBL inhibitor whilst the antibiotic agent is provided separately, or the compound of the invention may be formulated with the antibiotic agent whilst the SBL inhibitor is provided separately, or the SBL inhibitor may be formulated with the antibiotic agent whilst the compound of the invention is provided separately; or the compound of the invention, the SBL inhibitor and the antibiotic agent may each be formulated separately.

Preferably, the antibiotic agent which is administered with the compound of the invention, or used in the combinations or compositions of the invention, is a β-lactam antibiotic. More preferably, the antibiotic agent is a β-lactam antibiotic selected from carbapenems, penicillins, cephalosporins and penems. Examples of carbapenem antibiotics include Imipenem, Meropenem, Ertapenem, Doripenem and Biapenem. Examples of penicillins include Amoxicillin, Ampicillin, Ticarcillin, Piperacillin and Cloxacillin. Examples of cephalosporins include Cefazolin, Ceftriaxone, Ceftazidine and Ceftobiprole. Examples of penems include Faropenem. Other antibiotic agents include tobramycin, neomycin, streptomycin, gentamycin, tazobactam, rifampicin, ciprofloxacin, amikacin, colistin, aztreonam and levofloxacin. Preferably, the β-lactam antibiotic is a carbapenem antibiotic, more preferably imipenem or meropenem, most preferably meropenem.

Where an SBL inhibitor is present in the combinations or compositions of the invention, or is administered with a compound of the invention, the SBL inhibitor may be a compound of Formula (II) or a pharmaceutically acceptable salt thereof, 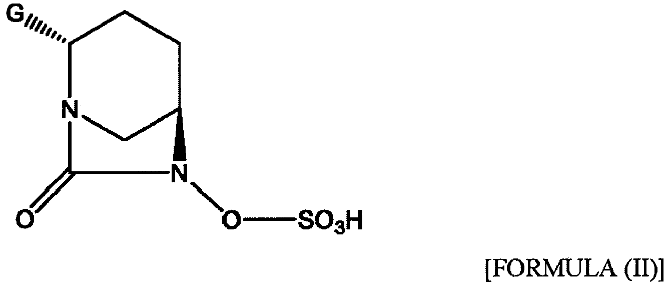 wherein
∘ G is selected from -CN and -C(O)NR^{j}R^{k};
∘ R^{k} is selected from -W and -Q-W; wherein W is selected from 5- to 6-membered heterocyclyl, R^{j} and -N(R^{j})₂; and Q is selected from -NR^{j}C(O)-, -C(O)-NR^{j}-, C₁₋₃ alkylene, -O-C₁₋₃ alkylene and -N(R^{j})-C₁₋₃ alkylene;
∘ each R^{j} is selected from H and unsubstituted C₁₋₃ alkyl, preferably H.

In Formula (II), when W is a 5- to 6-membered heterocyclyl, W is preferably a 6-membered heterocycle containing a nitrogen atom; more preferably W is piperidinyl. Preferably, in Formula (II), W is selected from 5- to 6-membered heterocyclyl and -N(R^{j})₂, more preferably W is selected from piperidinyl and NH₂. In formula (II), Q is preferably selected from - NR^{j}C(O)- and -O-C₁₋₃ alkylene. Preferably, in Formula (II), each R^{j} is H. Thus, preferred definitions G in formula (II) are -CN and -C(O)NHR^{k}, wherein R^{k} is selected from -W and - Q-W; wherein W is selected from 5- to 6-membered heterocyclyl, preferably pyridinyl, and - NH₂; and Q is selected from -NHC(O)- and -O-C₁₋₃ alkylene.

More preferably, in the pharmaceutical combination of the invention, the SBL inhibitor is selected from WCK4234, avibactam, relebactam, zidebactam and nacubactam, or pharmaceutically acceptable salts thereof. The structures of WCK4234, avibactam, relebactam, zidebactam and nacubactam are shown below. Such SBL inhibitors are commercially available and/or can be synthesized according to published protocols available to those skilled in the art. For example, WCK4234 and its synthesis is described in WO 2013/038330 and WO 2015/114595. Avibactam and its synthesis is described in Ball, M. et al, Org. Process Res. Dev., 2016, 20 (10), pp 1799-1805; and US 2012/323010. Relebactam and its synthesis is described in WO 2009/091856. Zidebactam and its synthesis is described in WO 2015/110885. Nacubactam and its synthesis is described in WO 2014/091268 and US 2016/272641.

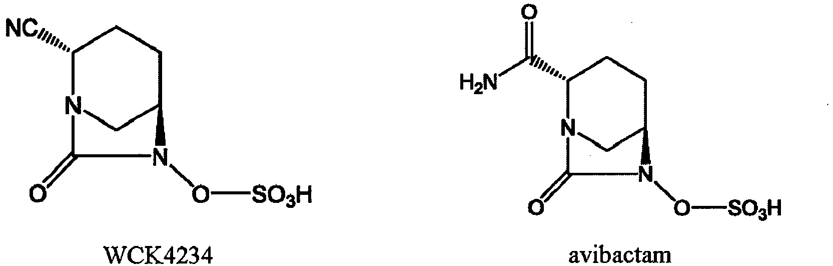

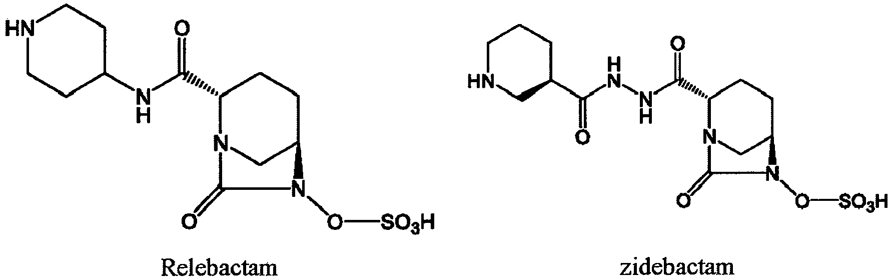

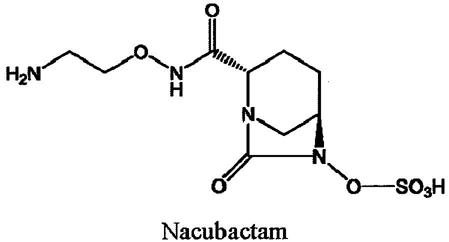

Most preferably, therefore, where an SBL inhibitor and/or an antibiotic agent are used in the present invention, the SBL inhibitor is selected from WCK4234, avibactam, relebactam, zidebactam and nacubactam, and pharmaceutically acceptable salts thereof; and the antibiotic agent is a carbapenem antibiotic, preferably meropenem.

The compounds, compositions or combinations described herein may be administered in a variety of dosage forms. Thus, they can be administered orally, for example as tablets, troches, lozenges, aqueous or oily suspensions, dispersible powders or granules. They may also be administered parenterally, whether subcutaneously, intravenously, intramuscularly, intrasternally, transdermally or by infusion techniques. The compound, composition or combination may also be administered as a suppository. Preferably, the compound, composition or combination may be administered via inhaled (aerosolised) or intravenous administration, most preferably by inhaled (aerosolised) administration.

The compounds, compositions or combinations described herein are typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

The compounds, compositions or combinations described herein may be formulated for inhaled (aerosolised) administration as a solution or suspension. The compounds, compositions or combinations described herein may be administered by a metered dose inhaler (MDI) or a nebulizer such as an electronic or jet nebulizer. Alternatively, the compounds, compositions or combinations described herein may be formulated for inhaled administration as a powdered drug, such formulations may be administered from a dry powder inhaler (DPI). When formulated for inhaled administration, the compound, composition or combination of the invention may be delivered in the form of particles which have a mass median aerodynamic diameter (MMAD) of from 1 to 100 µm, preferably from 1 to 50 µm, more preferably from 1 to 20 µm such as from 3 to 10 µm, e.g. from 4 to 6 µm. When the compound, composition or combination is delivered as a nebulized aerosol, the reference to particle diameters defines the MMAD of the droplets of the aerosol. The MMAD can be measured by any suitable technique such as laser diffraction.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

### Therapeutic Efficacy

The compounds of the present invention are therapeutically useful. The present invention therefore provides compounds, compositions and combinations as described herein, for use in medicine. The present invention provides compounds as described herein, for use in treating the human or animal body. For the avoidance of doubt, the compound of the invention may be administered in the form of a solvate.

The compounds, compositions and combinations of the invention are useful in treating or preventing bacterial infection. The present invention therefore provides a compound, composition or combination of the invention for use in medicine, in particular for use in treating or preventing bacterial infection. The invention also provides the use of a compound, composition or combination of the invention in the manufacture of a medicament for use in the prevention or treatment of bacterial infection. The invention also provides a method of treating or preventing bacterial infection in a subject in need thereof, which method comprises administering to said subject an effective amount of a compound, composition or combination as described herein.

The compounds of the invention may be used as standalone therapeutic agents. For example, the compounds of the invention may be used as standalone adjuncts in antibacterial therapy, for example in chemotherapy regimes. Alternatively, they may be used in combination with antibiotic agents, and optionally SBL inhibitors, to enhance the action of the antibiotic agent. The compounds of the invention may find particular use in treating or preventing bacterial infection caused by bacteria which are resistant to treatment with antibiotic agents when administered alone, particularly where the resistance is caused by presence of metallo-β-lactamase and/or serine-β-lactamase enzymes. Treatment or prevention of such infection with β-lactam antibiotics alone may be unsuccessful. The compounds are therefore useful in the removal or reduction of antibiotic resistance, in particular in Gram-negative bacteria.

Therefore, also provided is a compound of the invention for use in treating or preventing bacterial infection by co-administration with (i) an antibiotic agent and/or (ii) an SBL inhibitor. Also provided is an antibiotic agent for use in treating or preventing bacterial infection by co-administration with (i) a compound of the invention and optionally (ii) an SBL inhibitor. Also provided is an SBL inhibitor for use in treating or preventing bacterial infection by co-administration with (i) a compound of the invention and optionally (ii) an antibiotic agent.

Also provided is the use of a compound of the invention in the manufacture of a medicament for use in treating or preventing bacterial infection by co-administration with (i) an antibiotic agent and/or (ii) an SBL inhibitor. Also provided is the use of an antibiotic agent in the manufacture of a medicament for use in treating or preventing bacterial infection by co-administration with (i) a compound of the invention and optionally (ii) an SBL inhibitor. Also provided is the use of an SBL inhibitor in the manufacture of a medicament for use in treating or preventing bacterial infection by co-administration with (i) a compound of the invention and optionally (ii) an antibiotic agent.

Also provided is a method of treating or preventing bacterial infection in a subject in need thereof, which method comprises co-administering a compound of the invention with (i) and antibiotic agent and/or (ii) an SBL inhibitor.

In one aspect, the subject to be treated is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

The compounds, compositions and combinations described herein are useful in the treatment of bacterial infection which occurs after a relapse following an antibiotic treatment. The compounds, compositions and combinations can therefore be used in the treatment of a patient who has previously received antibiotic treatment for the (same episode of) bacterial infection.

The bacterium causing the infection may be any bacterium expressing a metallo- β-lactamase enzyme or an analogue thereof. Typically the bacterium causing the infection expresses a MBL enzyme. The bacterium is typically Gram-negative. The bacterium may in particular be a pathogenic bacterium. Typically, the bacterial infection to be treated using the compounds of the invention is resistant to treatment with a conventional antibiotic when the conventional antibiotic is used alone.

The Gram-negative bacteria of which antibiotic resistance can be removed using the compounds of general formula (I) are bacteria which produce metallo-β-lactamases, which may be metallo-β-lactamases of subclasses B1, B2 or B3, for example IMP-type (including IMP-1), VIM-type (including VIM-1 and VIM-2) and NDM-type (including NDM-1) enzymes. Typically, the Gram-negative bacteria express NDM-type MBL enzymes, VIM-type MBL enzymes and/or IMP-type MBL enzymes; more typically the bacteria express NDM-type MBL enzymes and/or VIM-type MBL enzymes; most typically the bacteria express NDM-type MBL enzymes. The Gram-negative bacteria may express one or more of the following enzymes: ACT-TYPE, CMY-4, CTX-M-3, CTX-M-15, IMP-1, IMP-28, KPC-2, NDM-1, OXA-48, OXA-181, SHV-OSBL, SHV-11, SHV-12, TEM-OSBL, TEM-1, VIM-1, and/or VIM-19.

The bacterial infection may be caused by bacteria from the families Enterobacteriaceae, Pseudomonadaceae and/or Moraxellaceae, more typically the bacterial infection is caused by bacteria from the families Enterobacteriaceae and/or Pseudomonadaceae, and most typically the bacterial infection is caused by bacteria from the family Enterobacteriaceae. The bacterial infection may be caused by *Pseudomonas* (e.g. *Pseudomonas aeruginosa, Pseudomonas oryzihabitans, or Pseudomonas plecoglossicida*), *Klebsiella, Escherichia, Acinetobacter* or *Burkholderia.* For example, the bacterial infection may be caused by *Klebsiella pneumonia, Escherichia coli, Pseudomonas aeruginosa, Burkholderia cepacia* or *Acinetobacter baumannii.* The bacterial infection may be caused by *Escherichia coli, Klebsiella pneumonia,* or *Klebsiella oxytoca.* The bacterium may be an opportunistic pathogen.

The compounds, compositions and products of the invention are useful in the prevention or treatment of infection by the following strains:
NTBC020 (*E. coli* strain expressing NDM-1, TEM-1 and CTX-M-15); NTBC035-2 (*K. pneumoniae* strain expressing NDM-1, CMY-4 and SHV-11); NTBC104-1 (*K. pneumoniae* strain expressing NDM-1 and SHV-11); NTBC123 (*K. pneumoniae* strain expressing NDM-1); NTBC018 (*Citrobacter freundii* expressing VIM-2) ; NTBC062 (*K. pneumoniae* strain expressing IMP-1 and TEM-1); NTBC024 (*K. pneumoniae* strain expressing VIM-19, TEM-1 and CTX-M-3); NTBC042 (*E. coli* strain expressing VIM-1, TEM-1 , CTX-M-15, SHV-12); NTBC055 (*E. Coli* strain expressing VIM-1); and NTBC039 (*K. oxytoca* strain expressing IMP-28).

The compounds, compositions and products of the invention may also be useful in the prevention or treatment of infection by the following strains. The triple combination is particularly useful in the prevention or treatment of infection by these strains:
NTBC019 (*K. pneumonia* strain expressing NDM-1, CTX-M-15 and OXA-181); NTBC185 (*K. pneumonia* strain expressing SHV-OSBL, TEM-OSBL, NDM-1 and OXA-48);
NTBC186 (*K. pneumonia* strain expressing ACT-TYPE, VIM-1 and OXA-48); NTBC187 (*K. pneumonia* strain expressing SHV-OSBL, NDM-1 and OXA-48); and
NTBC188 (*K. pneumonia* strain expressing NDM-1 and KPC-2).

The compound, composition or combination of the invention may be used to treat or prevent infections and conditions caused by any one or a combination of the above-mentioned bacteria. In particular, the compound, composition or combination of the invention may be used in the treatment or prevention of pneumonia. The compound, composition or combination may also be used in the treatment of septic shock, urinary tract infection, and infections of the gastrointestinal tract, skin or soft tissue.

The compound, composition or combination of the invention may be used to treat patients with Carbapenem Resistant Enterobacteriaceae (CRE). CRE can be found in the community or in hospitals and other institutions which are commonly associated with long term patients and those that are undergoing significant medical interventions such as are commonly cared for in Intensive Care Units (ICUs).

A compound, composition or combination of the invention can be administered to the subject in order to prevent the onset or reoccurrence of one or more symptoms of the bacterial infection. This is prophylaxis. In this embodiment, the subject can be asymptomatic. The subject is typically one that has been exposed to the bacterium. A prophylactically effective amount of the agent or formulation is administered to such a subject. A prophylactically effective amount is an amount which prevents the onset of one or more symptoms of the bacterial infection.

A compound, composition or combination of the invention can be administered to the subject in order to treat one or more symptoms of the bacterial infection. In this embodiment, the subject is typically symptomatic. A therapeutically effective amount of the agent or formulation is administered to such a subject. A therapeutically effective amount is an amount effective to ameliorate one or more symptoms of the disorder.

A therapeutically or prophylactically effective amount of the compound of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 70 mg to 3.5 g.

When the compound of the invention is administered to a subject in combination with another active agent (for example in the form of a pharmaceutical combination comprising an antibiotic agent and optionally an SBL inhibitor), the dose of the other active agent (e.g. SBL inhibitor and/or antibiotic agent) can be determined as described above. The dose may be determined according to various parameters, especially according to the agent used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 70 mg to 3.5 g.

The following Examples illustrate the invention. They do not however, limit the invention in any way. In this regard, it is important to understand that the particular assay used in the Examples section is designed only to provide an indication of biological activity. There are many assays available to determine biological activity, and a negative result in any one particular assay is therefore not determinative.

### General synthetic methodology

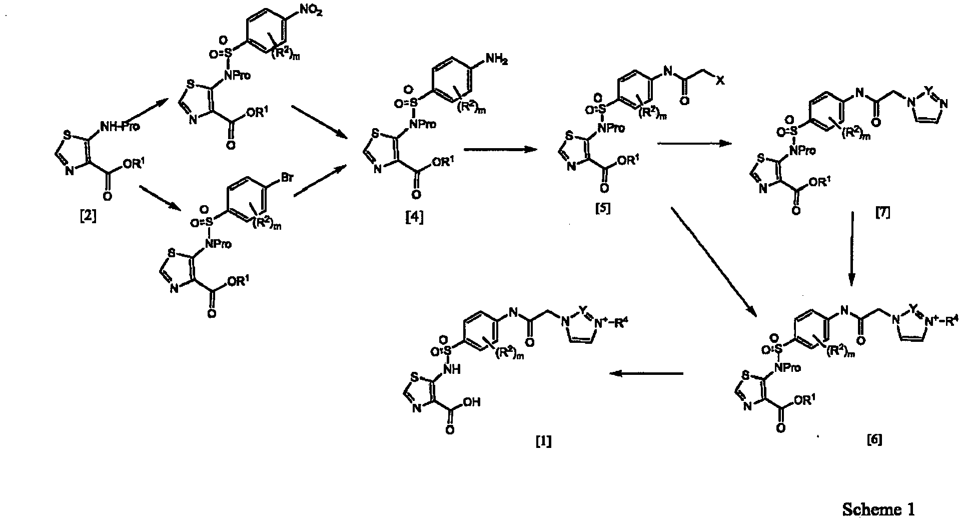

The synthesis of azolium compounds of formula (1) (Scheme 1) can be achieved by a number of different chemical syntheses. For example anilines (4) are available by reaction of aminothiazoles (2) (where Pro is H or a protecting group such as p-methoxybenzyl and R1 is an ester such as lower alkyl or t-butyl) with arylsulphonyl chlorides (3) containing a nitro group followed by standard reduction of nitro to amino giving anilines (4). Alternatively reaction of (2) with arylsulphonyl chlorides containing, for example, a bromine atom on the aryl ring followed by standard Buchwald amination can access the same anilines (4). Suitable nucleophiles for the Buchwald amination include ammonia and benzophenone imine followed by hydrolysis.
**Route-1** From the anilines (4), reaction with for example chloroacetyl chloride gives key chloroacetamide (5) which can be reacted with various substituted azoles to give quaternised heterocycles (6) in protected form, deprotection of which (eg using TFA in the case where R1 = t-butyl and Pro = p-methoxybenzyl) then reveals compounds (1).
**Route-2** Alternatively reaction of the key chloroacetamide (5) with the analogous NH azole affords the corresponding azoles (7). Alkylation with the appropriate alkyl halide then generates the quaternised heterocycle (1) in protected form as before and deprotection of which then affords azolium compounds (1).

### Examples

1H NMR spectra are reported at 300, 400 or 500 MHz in DMSO-d6 solutions (δ in ppm), using chloroform as the reference standard (7.25 ppm). When peak multiplicities are reported, the following abbreviations are used: s (singlet), d (doublet), t (triplet), m (multiplet), bs (broadened singlet), dd (doublet of doublets), dt (doublet of triplets), q (quartet). Coupling constants, when given, are reported in hertz (Hz).

### Abbreviations

- ACN: Acetonitrile
- AcOH: Acetic acid
- AIBN: Azobisisobutyronitrile
- Cs₂CO₃: Cesium carbonate
- cfu: Colony forming unit
- CuI: Copper iodide
- DCM: Dichloromethane
- DEA: Diethylamine
- DIPEA: N,N-Diisopropylethylamine
- DMAP: 4-Dimethylaminopyridine
- DMF: Dimethylformamide
- DMSO: Dimethyl sulfoxide
- dppf: 1,1'-Bis(diphenylphosphino)ferrocene
- EDC.HCl: N-(3-Dimethylaminopropyl)-N'-ethylcarbodiimide hydrochloride
- EtOAc: Ethyl acetate
- EtOH: Ethanol
- Et3N: Triethylamine
- FA: Formic acid

- HATU: 1-[Bis(dimethylamino)methylene]-1H-1,2,3-triazolo[4,5-b]pyridinium 3-oxid hexafluorophosphate

- HCl: Hydrochloric acid
- HOBt: Hydroxybenzotriazole
- H2SO4: Sulfuric Acid
- IPA: *Iso*-propyl alcohol
- Km: Michaelis constant
- MeI: Methyl iodide
- MeOH: Methanol
- NBS: N-bromo succinimide
- Na2CO3: Sodium carbonate
- Na2SO4: Sodium sulfate
- Pd₂(dba)₃: Tris(dibenzylideneacetone)dipalladium(0)
- PdCl₂(PPh3)2: Bis(triphenylphosphine)palladium(II) dichloride
- PdCl₂(dppf): [1,1'-Bis(diphenylphosphino)ferrocene]dichloropalladium(II)
- PMB: Paramethoxybenzyl
- TEA: Triethylamine
- TES: Triethylsilane
- TMSOTf: Trimethylsilyl trifluoromethanesulfonate
- TFA: Trifluoroacetic acid
- THF: Tetrahydrofurane
- T3P: Propylphosphinic anhydride
- RT: Room temperature

Formula 2 below depicts the structure of (RuPhos) Palladium (II) phenethylamine chloride (1:1 MTBE adduct) used for Buchwald coupling steps (RuPhos Pd G1 complex).

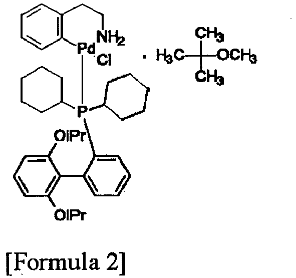

### Tert-butyl5-[(4-methoxyphenyl)methylamino]thiazole-4-carboxylate

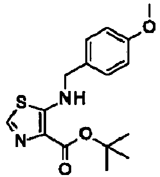

A suspension of potassium tert-butoxide (874 mg, 7.79 mmol) in dry tetrahydrofuran (10 mL) was stirred vigorously at room temperature. To this, a solution of tert-butyl isocyanoacetate (1.0 g, 7.08 mmol) in dry tetrahydrofuran (5 mL) was added drop wise and the mixture stirred at room temperature for 10 minutes. To this, a solution of 4-methoxybenzyl isothiocyanate (1.27 g, 7.08 mmol) in dry tetrahydrofuran (5 mL) was added drop wise at room temperature. After 2 hours the solution was poured into saturated NaHCO3 solution and extracted with ethyl acetate. The organic layer was dried with Na2SO4, filtered and concentrated in vacuo to dryness. The residue was purified by silica gel chromatography (eluting with 0-50% ethyl acetate/cyclohexane) affording the title product as a pale yellow solid (852 mg).
1H NMR (CDCl₃) δ: 7.81 (1H, m), 7.73 (1H, br s), 7.31-7.23 (2H, m), 6.92-6.85 (2H, m), 4.35 (2H, d), 3.80 (3H, s), 1.61 (9H, s).
M/z 321 (M+H)⁺

### Key Intermediate 1 tert-butyl 5-[[4-[(2-chloroacetyl)amino]-3-fluoro-phenyl]sulfonyl-[(4-methoxyphenyl)methyl]amino]thiazole-4-carboxylate

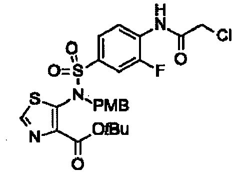

### a. tert-butyl 5-[(3-fluoro-4-nitro-phenyl)sulfonyl-[(4-methoxyphenyl)methyl]amino]thiazole-4-carboxylate

A solution of tert-butyl 5-[(4-methoxyphenyl)methylamino]thiazole-4-carboxylate (15 g, 46.8 mmol) in THF (150 mL) was added to NaH (9.3 g, 234 mmol) suspension in THF (150 mL) at 0 °C under argon atmosphere. The reaction mixture was stirred at RT for 30 minutes. Then a solution of 3-fluoro-4-nitro-benzenesulfonyl chloride (12.3 g, 51.4 mmol) in THF (150 mL) was added at 0 °C under argon atmosphere. The resulting reaction mixture was stirred at RT for 1 h, added to ice cold water (600 mL) and extracted with ethyl acetate (2 x 300 mL). The combined organic layer was dried over Na₂SO₄, filtered and concentrated. The crude material was purified by trituration with diethyl ether (2 x 100 mL) to afford a yellow solid (17 g, 69%).
M/z 524.1 (M+H)⁺

### b. tert-butyl 5-[(4-amino-3-fluoro-phenyl)sulfonyl-[(4-methoxyphenyl)methyl]amino]thiazole-4-carboxylate

Na₂S₂O₄ (49.8 g, 286.5 mmol) was added to a stirred solution of tert-butyl 5-[(3-fluoro-4-nitro-phenyl)sulfonyl-[(4-methoxyphenyl)methyl]amino]thiazole-4-carboxylate (15 g, 28.6 mmol) in THF: H₂O (1:1, 200 mL) at RT. The resulting reaction mixture was stirred for 3 h at the same temperature, diluted with water (500 mL) and extracted with EtOAc (2 x 300 mL). The organic layer was washed brine solution (100 mL), dried over Na₂SO₄, filtered and concentrated under reduced pressure. The crude material was purified by trituration with diethyl ether (2 x 100 mL) to afford a yellow solid (12 g, 85%).
M/z 494.1 (M+H)⁺

### c. tert-butyl 5-[[4-[(2-chloroacetyl)amino]-3-fluoro-phenyl] sulfonyl-[(4-methoxyphenyl)methyl]amino]thiazole-4-carboxylate

Et₃N (276 mg, 2.73 mmol) and chloroacetyl chloride (185 mg, 1.64 mmol) were added to a stirred solution of tert-butyl 5-[(4-amino-3-fluoro-phenyl)sulfonyl-[(4-methoxyphenyl)methyl]amino]thiazole-4-carboxylate (450 mg, 0.91 mmol) in DCM (5 mL) at 0 °C. The resulting reaction mixture was stirred at RT for 2 h, quenched with ice cold water (5 mL) and extracted with DCM (2 x 10 mL). The combined organic layer was dried over Na₂SO₄. filtered and concentrated under reduced pressure. The crude material was purified by trituration with diethyl ether (2 x 5 mL) to afford Key Intermediate-1 as a green solid (400 mg, crude).
M/z 570.69 (M+H)⁺

### Key Intermediate 2 tert-butyl 5-[[4-[(2-chloroacetyl)amino]-3,5-difluoro-phenyl]sulfonyl-[(4-methoxyphenyl)methyl]amino]thiazole-4-carboxylate

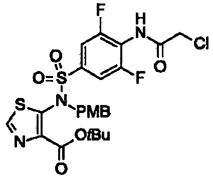

### a. tert-butyl 5-[(4-bromo-3,5-difluoro-phenyl)sulfonyl-[(4-methoxyphenyl)methyl]amino]thiazole-4-carboxylate

A solution of tert-butyl 5-[(4-methoxyphenyl)methylamino]thiazole-4-carboxylate (1 g, 3.12 mmol, 1 eq) in THF (15 mL) was added to NaH suspension in THF (10 mL) at 0 °C under argon atmosphere. After 30 minutes, a solution of 4-bromo-3,5-difluoro-benzenesulfonyl chloride (1.0 g, 3.43 mmol, 1.1 eq) in THF (15 mL) was added at 0 °C under argon atmosphere. The resulting reaction mixture was stirred at RT for 1 h, quenched with ice cold water (20 mL) and extracted with ethyl acetate (2 x 20 mL). The combined organic layer was dried over Na₂SO₄, filtered and concentrated. The crude material was purified by trituration with diethyl ether (2 x 5 mL) affording a pale yellow solid (800 mg, 44%).
M/z 577.0 (M+H)⁺

### b. tert-butyl 5-[(4-amino-3,5-difluoro-phenyl)sulfonyl-[(4-methoxyphenyl)methyl]amino]thiazole-4-carboxylate

A saturated solution of NH₃ in dioxane (120 mL) was added to a mixture of tert-butyl 5-[(4-bromo-3,5-difluoro-phenyl)sulfonyl-[(4-methoxyphenyl)methyl]amino]thiazole-4-carboxylate (2 g, 3.47 mmol), Xantphos (0.6 g, 1.04 mmol), Pd₂(dba)₃ (0.317 g, 0.34 mmol) and K₃PO₄ (2.2 g, 10.4 mmol). The resulting mixture was stirred in sealed tube at 100 °C for 5 h, filtered through Celite pad and the pad was washed with ethyl acetate (2 x 25 mL). The filtrate was concentrated under reduced pressure. The crude material was purified by flash chromatography (Combiflash system, eluting with 50% ethyl acetate in petroleum ether) affording a pale yellow solid (1.25 g, 70%).
M/z 512.4 (M+H)⁺ ; 534.56 (M+Na)⁺

### c. tert-butyl 5-[[4-[(2-chloroacetyl)amino]-3,5-difluoro-phenyl]sulfonyl-[(4-methoxyphenyl)methyl]amino]thiazole-4-carboxylate (Key Intermediate-2)

To a stirred solution of tert-butyl 5-[(4-amino-3,5-difluoro-phenyl)sulfonyl-[(4-methoxyphenyl)methyl]amino]thiazole-4-carboxylate (1.0 g, 1.95 mmol) in DCM (15 mL) was added Et₃N (0.82 mL, 3.90 mmol), DMAP (24 mg, 0.19 mmol) at 0 °C under nitrogen atmosphere. After 10 minutes, chloroacetyl chloride (0.31 mL, 3.90 mmol) was added to the reaction mixture at 0 °C. The mixture was stirred at 0 °C for 4 hours then diluted with CH₂Cl₂ (10 mL), washed with saturated. NaHCO₃ (2 x 10 mL) and then brine (10 mL). The organic layer was dried over Na₂SO₄, filtered and concentrated to get crude compound which was triturated with diethyl ether (2 x 10 mL) to obtain Key Intermediate-1 as a pale brown solid (650 mg, 61%).
M/z 586.1 [M-H]⁺

### Example 1 1-[({4-[(4-carboxy-1,3-thiazol-5-yl)sulfamoyl]-2-fluorophenyl}carbamoyl)methyl]-3-methyl-1H-imidazol-3-ium

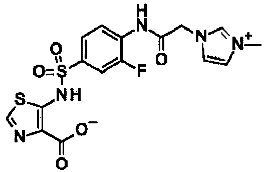

1-Methylimidazole (0.49 g, 6.05 mmol) was added to a stirred solution of Key Intermediate-1 (2.3 g, 4.03 mmol) in acetonitrile (25 mL) at RT. The reaction mixture was heated to 90 °C for 16 h and concentrated under reduced pressure. The crude material was triturated with diethyl ether (2 x 10 mL) to afford a brown solid (2.4g) which was used in the next steps without further purification.
M/z 616.38 (M)⁺
TFA: H₂O (9:1, 20 mL) was added to the brown solid (2.4 g) at RT. The reaction mixture was stirred for 3 h and concentrated under reduced pressure. The resulting crude product was triturated with diethyl ether (2 x 5 mL) and dried under high vacuum. The crude product was purified by preparative HPLC affording the title product as off-white solid (515 mg, 30% over 2 steps).
¹H NMR (300 MHz, DMSO-*d₆*) δ 13.40 (1H, brs), 10.61 (1H, brs), 9.08 (1H, s), 8.08 (1H, s), 8.06-8.04 (1H, m), 7.73-7.70 (2H, m), 7.56 (1H, s), 7.53 (1H, s), 5.27 (2H, s), 3.90 (3H, s).
M/z 440.1 [M]⁺

### LC-MS Conditions:

Column: BEH C18, 2.1*50 mm, 1.7 um;
Mobile phase: (A): 0.1% FA in water; Mobile phase: (B): 0.1% FA in ACN;
Gradient time/% of B: 0/3, 0.2/3, 1.5/98, 3/98, 3.1/3, 4/3;
Flow: 0.8 mL/min.

### Prep. HPLC Conditions:

Column used: PHENYL HEXYL (150*30) mm, 5 u;
Mobile phase: (A) 0.1% FA in water; Mobile Phase: (B) ACN;
Flow rate: 22 mL/min;
Gradient (T/%B): 0/5, 2/5, 4/20, 9/20, 9.1/99, 12/99, 12.1/5, 15/5;

### Example 2 1-[({4-[(4-carboxy-1,3-thiazol-5-yl)sulfamoyl]-2-fluorophenyl}carbamoyl)methyl]-2,3-dimethyl-1H-imidazol-3-ium

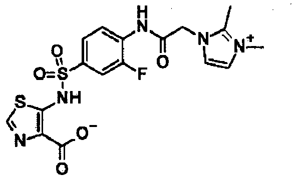

This was prepared by the same methodology as Example 1 except that 1,2-dimethylimidazole was used in place of 1-methylimidazole, affording the title compound as a pale brown solid (21 mg, 10% over 2 steps).
¹H NMR (400 MHz, DMSO-*d₆*): δ 13.41 (br s, 1 H), 10.57 (br s, 1 H), 8.08 (s, 1 H), 8.06-8.04 (m, 1 H), 7.65 (d, *J* = 2.4 Hz, 1 H), 7.62 (d, *J* = 2.4 Hz, 1 H), 7.54 (d, *J* = 8.8 Hz, 2 H), 5.22 (s, 2 H), 3.80 (s, 3 H), 2.54 (s, 3 H)
M/z 454.3 [M]⁺

### LC-MS Conditions:

Column: Acquity BEH C18 (50 mm x 2.1 mm, 1.7 um);
Mobile Phase: (A) 0.05% FA in water; Mobile Phase: (B) 0.05% FA in ACN
Time (min) /%B: 0/3, 0.4/3, 3.2/98, 3.8/98, 4.2/3, 4.5/3;
Column Temp: 35 °C; Flow Rate: 0.6 mL/min.

### Prep. HPLC Conditions:

Column: Atlantis T3 (250*19), 5 u;
Mobile Phase (A): 0.1% FA in water; Mobile Phase (B): 100% ACN
Gradient (T/% B): 0/5, 2/5, 6/30, 9.5/30, 9.6/99, 12/99, 12.1/5, 15/5;

### Example 3 2-amino-1-[({4-[(4-carboxy-1,3-thiazol-5-yl)sulfamoyl]-2-fluorophenyl}carbamoyl)methyl]-3-methyl-1H-imidazol-3-ium

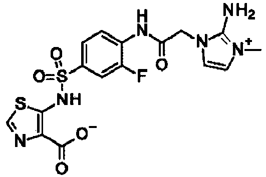

This was prepared by the same methodology as Example 1 except that 1-methyl-1H-imidazol-2-amine was used in place of 1-methylimidazole, affording the title compound as a pale brown solid (74 mg, 37% over 2 steps).
¹H NMR (400 MHz, DMSO-*d₆*): δ 13.42 (br s, 1 H), 10.43 (s, 1 H), 8.13-8.08 (m, 2 H), 7.83 (s, 2 H), 7.54-7.52 (m, 2 H), 7.04-7.02 (m, 2 H), 4.86 (s, 2 H), 3.47 (s, 3 H)
M/z 455.30 [M]⁺

### LC-MS Conditions:

Column: Acquity BEH C18 (50 mm x 2.1 mm, 1.7 um);
Mobile Phase: (A) 0.05% FA in water; Mobile Phase: (B) 0.05% FA in ACN;
Time (min) /%B: 0/5, 1.5/5, 3/15, 7/55, 10/95, 15/95, 17/5, 20/5;
Column Temp: 35 °C;
Flow Rate: 0.6 mL/min.

### Prep. HPLC Conditions:

Column used: Symmetry C18 (300*19) mm, 7 u;
Mobile phase: (A) 0.1% FA in water; Mobile Phase: (B) ACN
Flow: 19 mL/min;
Gradient (T/%B): 0/5, 1/5, 6/35, 8.2/35, 8.3/99, 10/99, 10.1/5, 13/5;

### Example 4 1-[(14-[(4-carboxy-1,3-thiazol-5-yl)sulfamoyl]-2-fluorophenyl}carbamoyl)methyl]-3-methyl-2-(methylsulianyl)-1H-imidazol-3-ium

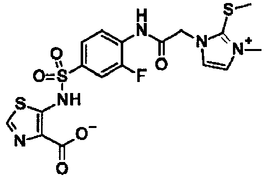

This was prepared by the same methodology as Example 1 except that 1-methyl-2-(methylsulfanyl)-1H-imidazole (commercially available from Combi-Blocks) was used in place of 1-methylimidazole and longer reaction time of 24 hours in step 1 was used, affording the title compound as a pale brown solid (12 mg, 8% over 2 steps).
¹H NMR (400 MHz, DMSO-*d₆*): δ 13.4 (br s, 1 H), 10.7 (s, 1 H), 8.08 (s, 1 H), 8.04 (t, *J* = 8.0 Hz, 1 H), 7.95 (d, *J* = 2.0 Hz, 1 H), 7.93 (d, *J* =2.0 Hz, 1 H), 7.56-7.53 (m, 2 H), 5.35 (s, 2 H), 3.96 (s, 3 H), 2.47 (s, 3 H)
M/z 486.22 [M]⁺

### LC-MS Conditions:

Column: Acquity BEH C18 (50 mm x 2.1 mm, 1.7 um);
Mobile Phase: (A) 0.05% FA in water; Mobile Phase: (B): 0.05% FA in ACN;
Time (min) /%B: 0/3, 0.4/3, 3.2/98, 3.8/98, 4.2/3, 4.5/3;
Column Temp: 35 °C;
Flow Rate: 0.6 mL/min.

### Prep. HPLC Conditions:

Column: Symmetry C18 (300*19) mm 7u;
Mobile phase: (A) 0.1% FA in water; Mobile phase: (B) ACN
Gradient (T/%B): 0/5, 1/5, 6/30, 12.10/30, 12.2/99, 14/99, 14.10/5, 17/5;
Flow: 19 mL/min;

### Example 5 1-[({4-[(4-carboxy-1,3-thiazol-5-yl)sulfamoyl]-2-fluorophenyl}carbamoyl)methyl]-1H,5H,6H,8H-4lambda5-imidazo[2,1-c][1,4]oxazin-4-ylium

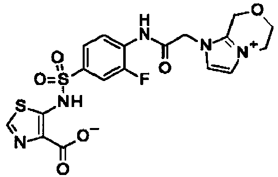

### (a) 5H,6H,8H-imidazo[2,1-c][1,4]oxazine

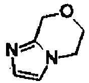

To a mixture of morpholin-3-one (500 mg, 4.94 mmol) and 2,2- diethoxyethan- 1- amine (1.3 g, 9.89 mmol) was added SnCl₄ (0.17 mL, 1.48 mmol) at RT. The resulting reaction mixture was heated to 150 °C for 4 h. The reaction mixture was quenched with water and the resulting precipitate was filtered off. The filtrate was evaporated under reduced pressure to give an oil (380 mg, 61%) which was used directly in the next step.
M/z 125.1 [M+H]⁺

### (b) 1-[({4-[(4-carboxy-1,3-thiazol-5-yl)sulfamoyl]-2-fluorophenyl}carbamoyl)methyl]-1H,5H,6H,8H-4lambda5-imidazo[2,1-c][1,4]oxazin-4- ylium

A mixture of 5H,6H,8H-imidazo[2,1-c][1,4]oxazine (130 mg, 1.1 mmol) and Key Intermediate-1 (400 mg, 0.7 mmol) in acetonitrile (5 mL) was heated at 80°C for 32 hours. The mixture was evaporated to dryness and redissolved in TFA:H2O (95:5, 5 mL). After 4 hours the mixture was evaporated and the residue purified by prep HPLC to afford the title compound as an off-white solid (86 mg, 25% over 2 steps).
¹H NMR (300 MHz, DMSO-*d₆*): δ 13.40 (br s, 1 H), 10.60 (br s, 1 H), 8.11-8.05 (m, 2 H), 7.77 (s, 2 H), 7.58-7.51 (m, 2 H), 5.16 (s, 2 H), 5.02 (s, 2 H), 4.29-4.20 (m, 2 H), 4.15-4.05 (m, 2 H).
M/z 482.2 [M]⁺

### LC-MS Conditions:

Column: Acquity BEH C18 (50 mm x 2.1 mm, 1.7 µm);
Mobile Phase: (A) 0.05% FA in water; Mobile Phase: (B) 0.05% FA in ACN
Gradient: Time (min)/ %B: 0/3, 0.4/3, 3.2/98, 3.8/98, 4.2/3, 4.5/3;
Column Temp: 35 °C;
Flow Rate: 0.6 mL/min.

### Prep. HPLC Conditions:

Column: Atlantis T3 (250*19) mm, 5 u;
Mobile phase: (A) 0.1% FA in water; Mobile phase: (B) ACN
Flow: 19 mL/min;
Gradient (T/%B): 0/5, 1/5, 7/35, 10.38/35, 10.9/99, 12/99, 12.1/5, 15/5;

### Examples 6 and 7 1-[({4-[(4-carboxy-1,3-thiazol-5-yl)sulfamoyl]-2-fluorophenyl}carbamoyl)methyl]-3,5-dimethyl-1H-imidazol-3-ium and 1-[({4-[(4-carboxy-1,3-thiazol-5-yl)sulfamoyl]-2-fluorophenyl}carbamoyl)methyl]-3,4-dimethyl-1H-imidazol-3-ium

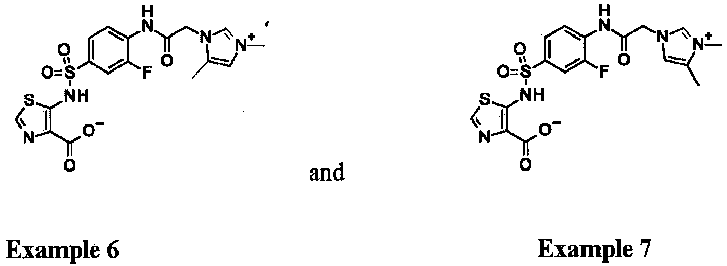

An equal mixture of 1,4-dimethylimidazole and 1,5-dimethylimidazole (prepared by non-selective methylation of 4-methylimidazole with methyl iodide and sodium hydroxide in methanol at room temperature for 5 hours followed by chromatography), (173 mg, 1.76 mmol) was combined with Key Intermediate-1 (1.0g, 1.76 mmol) in acetonitrile (10 ml) and heated at 80 °C for 16 hours then evaporated to give a brown oil which was purified twice by prep HPLC (conditions below):-

### Prep-HPLC conditions:

Column: Symmetry C18 (300*19) mm, 7 u;
Mobile phase: (A) 0.1% FA in water; Mobile Phase: (B) ACN
Flow: 19 mL/min;
Gradient (T/%B): 0/10, 1/10, 9.3/62, 9.35/99, 13/99, 13.01/10, 16/10;

### Preparative SFC Conditions

| | |
|---|---|
| Column: | Lux Cellulose-4 (250 x 30) mm, 5 µ; |
| % CO₂ | : 70.0% |
| % Co solvent | : 30.0% (0.5% DEA in Methanol); |
| Total Flow | : 60.0 g/min; |
| Back Pressure | : 100.0 bar; |
| UV | : 257 nm; |
| Stack time | : 12.3 min; |
| Load/Inj | : 18.0 mg; |

Purification gave as separate isomers 1-({[4-({4-[(tert-butoxy)carbonyl]-1,3-thiazol-5-yl}[(4-methoxyphenyl)methyl]sulfamoyl)-2-fluorophenyl]carbamoyl}methyl)- 3,4-dimethyl-1H-imidazol-3-ium chloride (110 mg) and 1-({[4-({4-[(tert-butoxy)carbonyl]-1,3-thiazol5-yl}[(4-methoxyphenyl)methyl]sulfamoyl)-2-fluorophenyl]carbamoyl}methyl)-3,5-dimethyl-1H-imidazol-3-ium chloride (100 mg). These intermediates were treated separately with TFA:H2O (95:5, 4 mL) at 0 °C and stirred at RT for 4 h. The mixtures were evaporated and the crude products individually subjected to purification by prep HPLC affording Example 6 (25 mg) and Example 7 (20 mg).

### Example 6

¹H NMR (400 MHz, DMSO-*d₆*) δ: 13.41 (s, 1 H), 10.55 (s, 1 H), 9.00 (s, 1 H), 8.08 (s, 1 H), 8.06-8.04 (m, 1 H), 7.54 (d, *J* = 8.8 Hz, 2 H), 7.48 (s, 1 H), 5.22 (s, 2 H), 3.79 (s, 3 H), 2.28 (s, 3H)
M/z 454.2 [M]⁺

### Example 7

¹H NMR (400 MHz, DMSO-*d₆*) δ: 13.42 (br s, 1 H), 10.61 (br s, 1 H), 9.01 (s, 1 H), 8.08 (s, 1 H), 8.05-8.01 (m, 1 H), 7.53 (d, *J* = 9.2 Hz, 2 H), 7.47 (s, 1 H), 5.20 (s, 2 H), 3.84 (s, 3 H), 2.21 (s, 3 H)
M/z 454.2 [M]⁺
The LC-MS and prep HPLC conditions for both compounds are as follows:-

### LC-MS Conditions:

Column: Acquity BEH C18 (50 mm x 2.1 mm, 1.7 um);
Mobile Phase: (A) 0.05% FA in water; Mobile Phase: (B) 0.05% FA in ACN
Time (min) /%B: 0/3, 0.4/3, 3.2/98, 3.8/98, 4.2/3, 4.5/3;
Column Temp: 35 °C;
Flow Rate: 0.6 mL/min.

### Prep. HPLC Conditions:

Column used: Atlantis T3 (250*19) mm, 5 u;
Mobile phase (A): 0.1% FA in water; Mobile phase: (B) ACN
Flow: 19 mL/min;
Gradient (T/%B): 0/5, 1/5, 9/70, 9.10/99, 11/99, 11.10/5, 14/5;

### Example 8 3-amino-1-[({4-[(4-carboxy-1,3-thiazol-5-yl)sulfamoyl]-2-fluorophenyl}carbamoyl)methyl]-2-methyl-1H-pyrazol-2-ium

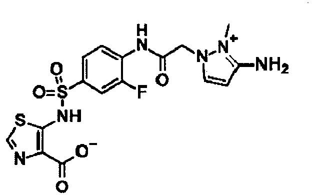

This was prepared by the same methodology as Example 1 except that 1- methyl- 1H-pyrazol- 5- amine was used in place of 1-methylimidazole, affording the title compound as a pale brown solid (80 mg, 33% over 2 steps).
¹H NMR (400 MHz, DMSO-*d₆*): δ 13.40 (br s, 1 H), 10.58 (s, 1 H), 8.08-8.03 (m, 3 H), 7.53 (d, *J* = 10.0 Hz, 2 H), 7.24 (s, 2 H), 5.87 (d, *J* = 3.2 Hz, 1 H), 5.25 (s, 2 H), 3.61 (s, 3 H)
M/z 455.2 [M]⁺

### LC-MS Conditions:

Acquity BEH C18 (50 mm x 2.1 mm, 1.7 µm);
Mobile phase: (A) 0.05% FA in water; Mobile phase: (B) 0.1% FA in ACN
Gradient: Time (min)/ %B: 0/3, 0.4/3, 3.2/98, 3.8/98, 4.2/3, 4.5/3;
Column Temp: 35 °C;
Flow Rate: 0.6 mL/min.

### Prep. HPLC Conditions:

Column:- X-BRIDGE C18 (150 x 30 mm); 5 u;
Mobile phase: (A) 0.1% FA in water; Mobile phase: (B) ACN
Gradient (T/%B): 0/5, 1/5, 6/20, 11.9/20, 12/99, 15/99, 15.1/5, 17/5;

Flow Rate: 19 mL/min;

### Example 9 1-[({4-[(4-carboxy-1,3-thiazol-5-yl)sulfamoyl]-2-fluorophenyl}carbamoyl)methyl]-3-methyl-1H-1,2,3-triazol-3-ium

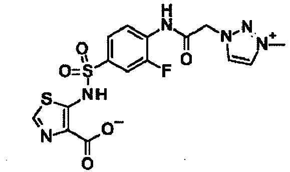

This was prepared by a variation of the methodology of Example 1, where the mixture was heated 100 °C for 90 minutes in a microwave apparatus and 1- methyl-1H-1,2,3-triazole was used in place of 1-methylimidazole, affording the title compound as a pale brown solid (155 mg, 20% over 2 steps).
¹H NMR (400 MHz, DMSO-*d₆*): δ 13.40 (br s, 1 H), 10.74 (s, 1 H), 8.90 (s, 1 H), 8.87 (s, 1 H), 8.08-8.03 (m, 2 H), 7.56-7.53 (m, 2 H), 5.77 (s, 2 H), 4.36 (s, 3 H)
M/z 440.1 [M]⁺

### LC-MS Conditions:

Acquity UPLC BEH C18 (50 mm x 2.1 mm, 1.7 µm);
Mobile phase: (A) 0.1% FA in ACN; Mobile phase: (B) 0.1% FA in water
Gradient: Time (min)/ %A: 0/2, 0.4/2, 2.2/98, 2.6/98, 2.61/2, 3.0/2;
Column Temp: 60 °C,
Flow Rate: 0.8 mL/min

### Prep. HPLC Conditions:

Column:- X-BRIDGE C18 (150 x 30 mm); 5 u
Mobile phase: (A) 0.05% FA in water; Mobile phase: (B) ACN
Gradient (T/%B): 0/5, 8/40, 8.1/98, 10/98, 10.1/5, 13/5;

Flow Rate: 25 mL/min;

### Example 10 1-[({4-[(4-carboxy-1,3-thiazol-5-yl)sulfamoyl]-2-fluorophenyl}carbamoyl)methyl]-2-methyl-1H-pyrazol-2-ium

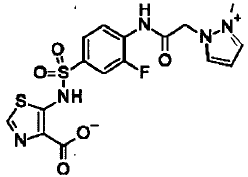

This was prepared by a variation of the methodology of Example 1 where the mixture was heated for 48 hours and 1- methylpyrazole was used in place of 1-methylimidazole, affording the title compound as a pale brown solid (34 mg, 12% over 2 steps),
¹H NMR (400 MHz, DMSO-*d₆*): δ 13.40 (br s, 1 H), 10.75 (s, 1 H), 8.56 (d, *J* = 2.4 Hz, 1 H), 8.54 (d, *J* = 2.4 Hz, 1 H), 8.08 (s, 1 H), 8.07-8.04 (m, 1 H), 7.54 (d, *J* = 8.8 Hz, 2 H), 6.91 (dd, *J* = 3.2 Hz, *J* = 2.8 Hz, 1 H), 5.63 (s, 2 H), 4.06 (s, 3 H).
M/z 439.3 [M]⁺

### LC-MS Conditions:

Column: Acquity BEH C18 (50 mm x 2.1 mm, 1.7 um);
Mobile phase: (A) 0.05% FA in water; Mobile phase: (B) 0.05% FA in ACN
Time (min) /%B: 0/3, 0.4/3, 3.2/98, 3.8/98, 4.2/3, 4.5/3;
Column Temp: 35 °C;
Flow Rate: 0.6 mL/min.

### Prep. HPLC Conditions:

Column: - X-BRIDGE C18 (150 x 30 mm); 5 u;
Mobile phase: (A) 0.1% FA in water; Mobile phase: (B) ACN
Gradient (T/%B): 0/5, 1/5, 6/20, 11.9/20, 12/99, 15/99, 15.1/5, 17/5;

Flow Rate: 19 mL/min;

### Example 11 3-(2-aminoethyl)-1-[({4-[(4-carboxy-1,3-thiazol-5-yl)sulfamoyl]-2-fluorophenyl}carbamoyl)methyl]-1H-imidazol-3-ium

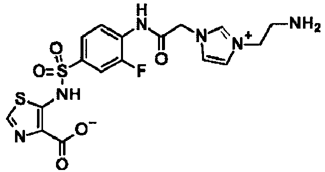

### a. tert-butyl N-(2-imidazol-1-ylethyl)carbamate

To a suspension of NaH (1.3 g, 33.4 mmol) in DMF (8 mL) was added a solution of imidazole (2.7 g, 40.1 mmol) in DMF (8 mL) at 0 °C under argon atmosphere. After 30 minutes, a solution of tert-butyl N-(2-bromoethyl)carbamate (3.0 g, 13.3 mmol) in DMF (8 mL) was added to the reaction mixture at 0 °C under argon atmosphere. The resulting reaction mixture was stirred at RT for 16 hours then poured into ice cold water (100 mL) and extracted with ethyl acetate (3 x 30 mL). The organic layer was washed with water (2 x 30 mL) and brine (2 x 30 mL). The combined organic layer was dried over Na₂SO₄, filtered and concentrated under reduced pressure to obtain tert-butyl N-(2-imidazol-1-ylethyl)carbamate as a brown oil (1.6g).
M/z 212.36 (M+H)⁺

### b. tert-butyl 5-[[4-[[2-[3-[2-(tert-butoxycarbonylamino)ethyl]imidazol-3-ium-1-yl]acetyl]amino]-3-fluoro-phenyl]sulfonyl-[(4methoxyphenyl)methyl]amino]thiazole-4-carboxylate

Tert-butyl N-(2-imidazol-1-ylethyl)carbamate (1.2g, 1.6 mmol) was added to a solution of Key Intermediate-1 (2.0g, 3.5 mmol) in CH₃CN (25 mL) at RT. The resulting reaction mixture was heated to 85 °C for 16 h, cooled to RT and more tert-butyl N-(2-imidazol-1-ylethyl)carbamate (0.4g, 1.8 mmol) was added. The resulting reaction mixture was heated to 85 °C for 8 h, cooled to RT and concentrated under reduced pressure. The crude material was triturated with diethyl ether (2 x 10 mL) and dried under high vacuum to afford a pale brown solid (2.5g).
M/z 745.51 [M]⁺

### c. 3-(2-aminoethyl)-1-[({4-[(4-carboxy-1,3-thiazol-5-yl)sulfamoyl]-2-fluorophenyl}carbamoyl)methyl]-1H-imidazol-3-ium

TFA (8 mL) was added to tert-butyl 5-[[4-[[2-[3-[2-(tert-butoxycarbonylamino)ethyl]imidazol-3-ium-1-yl]acetyl]amino]-3-fluoro-phenyl]sulfonyl-[(4 methoxyphenyl)methyl]amino]thiazole-4-carboxylate (1.25g). The reaction mixture was stirred at RT for 2 h and concentrated under reduced pressure. The resulting crude product was triturated with diethyl ether (2 x 10 mL) and dried under high vacuum to afford an off-white solid. This was purified by prep. HPLC to afford 3-(2-aminoethyl)-1-[({4-[(4-carboxy-1,3-thiazol-5-yl)sulfamoyl]-2-fluorophenyl}carbamoyl)methyl]-1H-imidazol-3-ium as a white solid (535 mg).
¹H NMR (500 MHz, DMSO-*d₆*): δ 13.4 (br s, 1 H), 10.6 (s, 1 H), 9.13 (s, 1 H), 8.11-8.06 (m, 2 H), 7.79-7.78 (m, 2 H), 7.56 (s, 1 H), 7.54 (s, 1 H), 7.50-6.80 (br s, 2 H), 5.30 (s, 2 H), 4.40 (t, *J* = 6.0 Hz, 2 H), 3.27 (t, *J* = 6.0 Hz, 2 H).
M/z 469.2 [M]⁺

### LC-MS Conditions:

Column - AQUITY UPLC BEH C18 (1.7 µm, 2.1 x 50 mm);
Mobile phase: (A) 0.1% FA in water; Mobile phase: (B) 0.1% FA in ACN
Gradient (T/%B): 0/97, 0.3/97, 2.2/2, 3.3/2, 4.5/2, 4.51/97;
Flow Rate: 0.6 mL/min;
Temperature: 35 °C.

### Prep. HPLC Conditions:

Column: X-BRIDGE-C18 (150*30), 5 u;
Mobile phase: (A) 0.1 % FA in water: Mobile phase: (B) ACN
Gradient: (T/%B):- 0/5, 1/5, 8/45, 8.1/98, 10/98, 10.1/5, 13/5;
Flow Rate: 25 mL/min.

### Example 11a 3-(2-Aminoethyl)-1-[({4-[(4-carboxylicacid-1,3-thiazol-5-yl)sulfamoyl]-2-fluorophenyl}carbamoyl)methyl]-1H-imidazol-3-ium chloride

3-(2-aminoethyl)-1-[({4-[(4-carboxy-1,3-thiazol-5-yl)sulfamoyl]-2-fluorophenyl}carbamoyl)methyl]-1H-imidazol-3-ium, prepared as in Example 11 (350 mg, 0.74 mmol) was dissolved in 0.1N aq HCl (22.3 mL, 2.23 mmol) affording a clear solution of pH3. The resulting reaction mixture was stirred at room temperature for 10 minutes and then lyophilized affording a white solid (330 mg, 81%).
¹H NMR (500 MHz, DMSO-*d₆*): δ 13.4 (br s, 1H), 10.84 (s, 1 H), 9.27 (s, 1 H), 8.55 (s, 1 H), 8.45 (br s, 3 H), 8.21 (t, *J* = 8.0 Hz, 1 H), 7.86 (dd, *J* = 2.0 Hz, *J* = 1.5 Hz, 1 H), 7.79 (dd, *J* = 2.0 Hz, *J =* 1.5 Hz, 1 H), 7.74 (dd, *J* = 10.5 Hz, *J* = 2.0 Hz, 1 H), 7.65 (dd, *J* = 8.0 Hz, *J* = 2.0 Hz, 1 H), 5.34 (s, 2 H), 4.56 (dd, *J* =6.0 Hz, *J* = 5.5 Hz, 2 H), 3.35-3.32 (m, 2 H).

### Example 12 3-(2-carbamimidamidoethyl)-1-[({4-[(4-carboxy-1,3-thiazol-5-yl)sulfamoyl]-2-fluorophenyl}carbamoyl)methyl]-1H-imidazol-3-ium

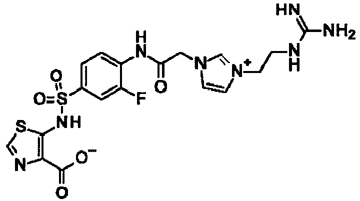

Pyrazole-l-carboxamidine, hydrochloride (94 mg) and DIPEA (0.23 mL) were added to a stirred solution of 3-(2-aminoethyl)-1-[({4-[(4-carboxy-1,3-thiazol-5-yl)sulfamoyl]-2-fluorophenyl}carbamoyl)methyl]-1H-imidazol-3-ium (Example 11), (200 mg in DMF (6 mL) at RT. The resulting reaction mixture was stirred at RT for 16 h and concentrated under reduced pressure. The crude product was purified by preparative HPLC affording the title product as an off-white solid (27 mg).
¹H NMR (500 MHz, DMSO-*d₆*) δ 13.41 (1H, brs), 9.16 (1H, s), 8.48 (1H, s), 8.07 (1H, s), 8.04 (1H, t, *J* = 8.0 Hz), 7.77 (2H, brs), 7.59-7.50 (4H, m), 5.24 (2H, s), 4.34 (2H, t, *J* = 6.0 Hz), 3.55 (2H, t, *J* = 6.0 Hz).
M/z 511.1 [M]⁺

### LC-MS Conditions:

Column: Acquity UPLC BEH C18 (50 mm x 2.1 mm, 1.7 um);
Mobile phase: (A) 0.1% FA in water; Mobile phase: (B) 0.1% FA in ACN;
Gradient (T/%B): 0/2, 0.2/2, 1.5/98, 2.6/98, 2.61/2, 3.2/2;
Column Temp: 45 °C;
Flow Rate: 0.8 mL/min.

### Prep. HPLC Conditions:

Column used: PHENYL HEXYL (150*30) mm, 5u;
Mobile phase: (A) 0.05% FA in water; Mobile phase: (B) ACN
Flow: 19 mL/min;
Gradient (T/%B): 0/2, 1/2, 9/36, 9.1/99, 12/99, 12.02/2, 15/2;

### Example 13 1-[({4-[(4-carboxy-1,3-thiazol-5-yl)sulfamoyl]-2,6-difluorophenyl}carbamoyl)methyl]-3-methyl-1H-imidazol-3-ium

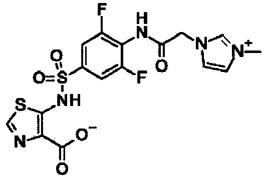

This was prepared by the same procedures as for Example 1 with the modification that Key Intermediate-2 was used in place of Key Intermediate-1, affording the title compound as an off-white solid (20 mg, 17% over 2 steps).
¹H NMR (500 MHz, DMSO-*d₆*): δ 13.24 (br s, 1 H), 10.54 (br s, 1 H), 9.10 (s, 1 H), 8.12 (s, 1 H), 7.73 (s, 1 H), 7.69 (s, 1 H), 7.43 (d, *J* = 7.0 Hz, 2 H), 5.31 (s, 2 H), 3.89 (s, 3 H)
M/z 458.1 [M]⁺

### LC-MS Conditions:

Column: Acquity UPLC BEH C18 (50 mm x 2.1 mm, 1.7 µm);
Mobile phase (A): 0.1% FA in water; Mobile phase: (B) 0.1% FA in ACN;
Gradient: Time(min)/ %B: 0/2, 0.2/2, 1.5/98, 2.6/98, 2.61/2, 3.2/2;
Column Temp: 45 °C;
Flow Rate: 0.8 mL/min.

### Prep. HPLC Conditions:

Column: KROMOSIL-C18 (150*25), 10 u;
Mobile phase (A): 0.05% FA in water; Mobile phase: (B) ACN
Gradient (T/%B):- 0/2, 2/2, 8/30, 9/30, 9.1/98, 11/98, 11.1/2, 13/2;

Flow Rate: 25 mL/min;

### Example 14 3-(2-aminoethyl)-1-[({4-[(4-carboxy-1,3-thiazol-5-yl)sulfamoyl]-2,6-difluorophenyl}carbamoyl)methyl]-1H-imidazol-3-ium

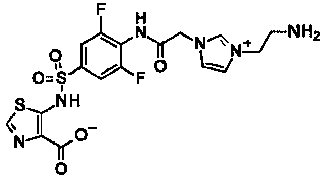

This was prepared by the same procedures as for Example 11 with the modification that Key Intermediate-2 was used in place of Key Intermediate-1 and a reaction time of 24 hours was used in step 1, affording the title compound as an off-white solid (140 mg, 22% over 2 steps).
¹H NMR (500 MHz, DMSO-*d₆*): δ 9.14 (s, 1 H), 8.14 (s, 1 H), 8.13 (s, 1 H), 7.75 (d, *J* = 1.5 Hz, 2 H), 7.45 (d, *J* = 7.0 Hz, 2 H), 5.34 (s, 2 H), 4.23 (t, *J* = 5.5 Hz, 2 H), 3.02 (t, *J* = 5.5 Hz, 2 H).
M/z 487.1 [M]⁺

### LC-MS Conditions:

Column: Luna Omegal.6 µm polar C18 100 A°, LC Column 50 x 2.1 mm;
Mobile phase: (A) 0.1% FA in water; Mobile phase: (B) 0.1% FA in ACN
Gradient: Time (min)/ %B: 0/2, 0.5/2, 3/98, 4/98, 4.5/2, 5/2;
Column Temp: 60 °C;
Flow Rate: 0.4 mL/min.

### Prep. HPLC Conditions:

Column: KROMOSIL- C18 (25*150 mm), 10 u;
Mobile phase: 0.05 % Formic Acid in H₂O: Acetonitrile
Gradient (T/%B): 0/2, 2/2, 7/30, 7.1/98, 10/98, 10.1/2, 12/2;
Flow Rate: 25 mL/ min;

### Example 15 1-(2-carbamimidamidoethyl)-3-[({4-[(4-carboxy-1,3-thiazol-5-yl)sulfamoyl]-2,6-difluorophenyl}carbamoyl)methyl]imidazol-1-ium

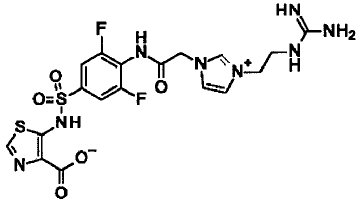

This compound was prepared in the same way as for Example 12 but starting from 2,6-difluorophenyl}carbamoyl)methyl]-1H-imidazol-3-ium (Example 14) affording the title compound as a white solid (47 mg, 17%).
¹H NMR (500 MHz, DMSO-*d₆*) δ 13.31 (br s, 1 H), 9.17 (s, 1 H), 8.42 (s, 1 H), 8.09 (s, 1 H), 7.76 (s, 1 H), 7.72 (s, 1 H), 7.60 (br s, 3 H), 7.30 (br s, 2 H), 5.13 (br s, 2 H), 4.32 (t, *J* = 5.5 Hz, 2 H), 3.54 (t, *J* = 5.5 Hz, 2 H)
M/z 529.1 [M]⁺

### LC-MS Conditions:

Column: Luna Omega1.6 µm polar C18 100 A°, LC Column 50 x 2.1 mm;
Mobile phase: (A) 0.1% FA in water; Mobile phase: (B) 0.1% FA in ACN
Gradient: Time (min)/ %B: 0/2, 0.5/2, 3/98, 4/98, 4.5/2, 5/2;
Column Temp: 60 °C;
Flow Rate: 0.4 mL/min.

### Prep. HPLC Conditions:

Column: KROMASIL- C18 (25mm*150), 10 u;
Mobile phase: (A) 0.05% FA in water: Mobile phase: (B) ACN
Gradient: (T/%B):- 0/5, 2/5, 7/30, 7.1/98, 9/98, 9.1/5, 11/5;
Flow Rate: 25 mL/min;

### Example 16 3-(3-aminopropyl)-1-[({4-[(4-carboxy-1,3-thiazol-5-yl)sulfamoyl]-2-fluorophenyl}carbamoyl)methyl]-1H-imidazol-3-ium

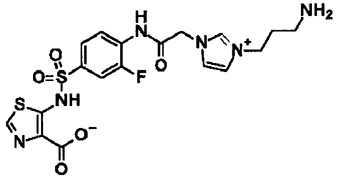

This was prepared by the same procedures as for Example 11 with the modification that tert-butyl N-(2-imidazol-1-ylpropyl)carbamate (prepared as in WO2008/67222) was used in place of tert-butyl N-(2-imidazol-1-ylethyl)carbamate, affording the title compound as an off-white solid (40 mg).
¹H NMR (500 MHz, DMSO-*d₆*): δ 9.18 (s, 1 H), 8.43 (s, 1 H), 8.08 (s, 1 H), 8.07 - 8.04 (m, 1 H), 7.80 (s, 1 H), 7.75 (s, 1 H), 7.55-7.53 (m, 2 H), 5.29 (s, 2 H), 4.30 (t, *J* = 7.0 Hz, 2 H), 2.58 (dd, *J* = 6.5 Hz, *J* = 6.0 Hz, 2 H), 1.95 - 1.89 (m, 2 H)
M/z 483.1 [M]⁺

### LC-MS Conditions:

Column: Acquity UPLC BEH C18 (50 mm x 2.1 mm, 1.7 µm);
Mobile phase: (A) 0.1% FA in water; Mobile phase: (B) 0.1% FA in ACN
Gradient: Time (min)/ %B: 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: Inertsil ODS-3 (20 *250 mm), 5.0 µm;
Mobile phase: (A) 0.1% FA in water; Mobile phase: (B) ACN
Gradient: (T%B):-1/3, 4/3, 12/45, 12.1/100;
Flow rate: 18.00 mL/min.

### Example 17 3-(3-carbamimidamidopropyl)-1-[({4-[(4-carboxy-1,3-thiazol-5-yl)sulfamoyl]-2-fluorophenyl}carbamoyl)methyl]-1H-imidazol-3-ium

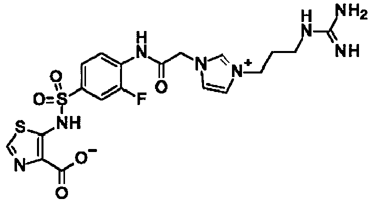

This was prepared from 3-(3-aminopropyl)-1-[({4-[(4-carboxy-1,3-thiazol-5-yl)sulfamoyl]-2-fluorophenyl}carbamoyl)methyl]-1H-imidazol-3-ium (Example 16) by the same procedure using pyrazole-l-carboxamidine as for the preparation of Example 12 giving the title compound as an off-white solid (110 mg).
¹H NMR (500 MHz, DMSO-*d₆*): δ 13.39 (br s, 1 H), 10.61 (br s, 1 H), 9.23 (s, 1 H), 9.00 (br s, 1 H), 8.07 (s, 1 H), 8.06 - 8.04 (m, 1 H), 7.83 (s, 1 H), 7.77 (s, 1 H), 7.60-7.53 (m, 6 H), 5.28 (s, 2 H), 4.28 (t, *J* = 7.0 Hz, 2 H), 3.12-3.05 (m, 2 H), 2.04-1.97 (m, 2 H)
M/z 425.2 [M]⁺

### LC-MS Conditions:

Column: Acquity UPLC BEH C18 (50 mm x 2.1 mm, 1.7 µm);
Mobile phase: (A) 0.1% FA in water, Mobile phase: (B) 0.1% FA in ACN
Gradient: Time (min)/ %B: 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: KROMOSIL-C18-(150*25); 10 u;
Mobile Phase: (A) 0.05% FA in water; Mobile phase: (B) ACN
Gradient (T/%B): 0/5, 2/5, 8/25, 8.5/25, 8.6/98, 11/98, 11.1/5, 13/5;

Flow Rate: 25 mL/min;

### Example 18 3-(2-aminoethyl)-1-[({4-[(4-carboxy-1,3-thiazol-5-yl)sulfamoyl]-2-cyanophenyl}carbamoyl)methyl]-1H-imidazol-3-ium

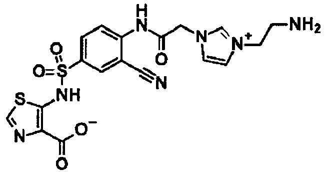

### (a) 4- bromo- 3- cyanobenzene- 1- sulfonyl chloride

To a solution of 5- amino- 2- bromobenzonitrile (2 g, 10.1 mmol) in AcOH (25 mL) was added conc. HCl (5 mL) at 0 °C. After 10 minutes a solution of NaNO₂ (0.77 g, 11.1 mmol) in H₂O (10 mL) at 0 °C and the stirred for 20 minutes at the same temperature.

Separately, SO₂ gas was purged into AcOH (25 mL) for 30 minutes, then a solution of CuCl₂ (1.6 g, 11.9 mmol) in H₂O (10 mL) was added at 0 °C and stirred for 20 minutes. This second solution was added to the first solution at 0 °C and the mixture stirred at 0 °C for 4 hours. The reaction mixture was quenched with ice cold water (20 mL). The resulting precipitate was filtered and dried under vacuum affording a white solid (1.2g, 42%)
M/z 259.9 [M-H]⁻

### (b) tert- butyl 5-{N-[(4-methoxyphenyl)methyl]4-bromo-3-cyanobenzenesulfonamido}-1,3-thiazole-4-carboxylate

A solution of tert- butyl 5-{[(4-methoxyphenyl)methyl]amino}-1,3-thiazole-4-carboxylate (1 g, 3.12 mmol) in THF (25 mL) was added to NaH (1.2 g, 31.2 mmol) suspension in THF (10 mL) at 0 °C under argon atmosphere. The reaction mixture was stirred at RT for 30 minutes. Then a solution of 4-bromo-3-cyanobenzene-1-sulfonyl chloride (1.3 g, 4.68 mmol) in THF (15 mL) was added to the above reaction mixture at 0 °C under argon atmosphere. The resulting reaction mixture was stirred at RT for 0.5 hour then poured into ice cold water (20 mL) and extracted with ethyl acetate (3 x 20 mL). The combined organic layer was dried over Na₂SO₄, filtered and concentrated to give an oil. This was purified by flash chromatography using 10-15% ethyl acetate in pet ether affording a pale brown solid (1.3g, 73%).
M/z 564.0 [M+H]⁺

### (c) tert- butyl 5- {N- [(4- methoxyphenyl)methyl]4- amino- 3-cyanobenzenesulfonamido}-1,3-thiazole-4-carboxylate

A mixture of tert- butyl 5-{N-[(4-methoxyphenyl)methyl]4-bromo-3-cyanobenzenesulfonamido}-1,3-thiazole-4-carboxylate (500 mg, 0.88 mmol), Xantphos (153 mg, 0.26 mmol), Pd₂(dba)₃ (81 mg, 0.08 mmol), K₃PO₄ (563 mg, 2.65 mmol) was treated with saturated NH₃ in dioxane (5 mL) and stirred in sealed tube at 80 °C for 8 hours. The reaction mixture was filtered through Celite pad and the pad was washed with ethyl acetate (2 x 10 mL). The combined filtrate was concentrated under reduced pressure to obtain an oil which was purified by flash chromatography using 5% MeOH in DCM affording a brown solid (220 mg, 49%).
M/z 499.1 [M-H]⁻

### (d) tert- butyl 5-{N-[(4-methoxyphenyl)methyl]4-(2-chloroacetamido)-3-cyanobenzenesulfonamido}-1,3-thiazole-4-carboxylate

To a solution of tert- butyl 5-{N-[(4-methoxyphenyl)methyl]4-amino-3-cyanobenzenesulfonamido}-1,3-thiazole-4-carboxylate (220 mg, 0.43 mmol) in DCM (5 mL) was added Et₃N (0.17 mL, 1.31 mmol) at RT. Then chloroacetyl chloride (0.06 mL, 0.79 mmol) was added at 0 °C and the reaction mixture was stirred at RT for 4 hours then washed with water and extracted with DCM (2 x 5 mL). The combined organic extracts were dried over Na₂SO₄, filtered and concentrated under reduced pressure to obtain an oil. This was purified by flash chromatography using 50% ethyl acetate in pet-ether affording a yellow solid (150 mg, 59%).
M/z 577.2 [M+H]⁺

### (e) 3-(2-aminoethyl)-1-[({4-[(4-carboxy-1,3-thiazol-5-yl)sulfamoyl]-2-cyanophenyl}carbamoyl)methyl]-1H-imidazol-3-ium

To a solution of tert-butyl5-{N-[(4-methoxyphenyl)methyl]4-(2-chloroacetamido)-3-cyanobenzenesulfonamido}-1,3-thiazole-4-carboxylate (150 mg, 0.25 mmol) in MeCN (5 mL) was added tert-butyl N-(2-imidazol-1-ylethyl)carbamate (71.3 mg, 0.33 mmol) at RT. The resulting reaction mixture was heated to 90 °C for 16 hours then evaporated under reduced pressure. The residue was triturated with diethyl ether (2 x 5 mL) and dried under high vacuum to obtain a brown solid (280 mg). This was treated with TFA (1 mL) at 0 °C and allowed to stir at RT for 3 hours. The mixture was evaporated under reduced pressure and the residue was triturated with diethyl ether (2 x 2 mL), *n*-pentane (2 x 2 mL) and dried under high vacuum. The crude product was purified by prep HPLC to afford a white solid (20 mg, 16% over 2 steps).
¹H NMR (400 MHz, DMSO-*d₆*): δ 9.12 (s, 1 H), 8.31 (br s, 1 H), 8.08 (s, 1 H), 7.97-7.92 (m, 2 H), 7.86-7.84 (m, 1 H), 7.72 (br s, 2 H), 5.14 (s, 2 H), 4.15 (t, *J* = 7.0 Hz, 2 H), 2.92 (t, *J* = 7.0 Hz, 2 H).
M/z 476.1 [M]⁺

### LC-MS Conditions:

Column: Acquity UPLC BEH C18 (50 mm x 2.1 mm, 1.7 µm);
Mobile Phase: (A) 0.1% FA in water; Mobile Phase: (B) 0.1% FA in ACN
Gradient (T/%B): 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: X-SELECT-CN-(250*19), 5 u;
Mobile phase: (A) 0.05% FA in water; Mobile phase: (B) ACN
Gradient: (T/%B): 0/5, 1/5, 8/40, 9/40, 9.1/98, 11/98, 11.1/5, 14/5;
Flow Rate: 18 mL/min;

### Example 19 3-(2-carbamimidamidoethyl)-1-[({4-[(4-carboxy-1,3-thiazol-5-yl)sulfamoyl]-2-cyanophenyl}carbamoyl)methyl]-1H-imidazol-3-ium

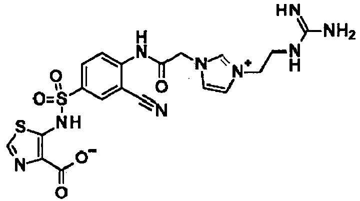

### (a) tert-butylN-[(1Z)-{[(tert-butoxy)carbonyl]amino}({[2-(1H-imidazol-1-yl)ethyl]amino})methylidene]carbamate

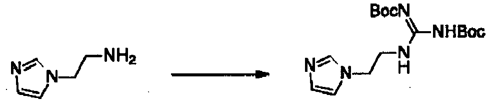

To a stirred solution of 2-(1H-imidazol-1-yl)ethan-1-amine dihydrochloride (commercially available from Sigma Aldrich) (600 mg, 3.25 mmol) in DMF (5 mL) was added DIPEA (2.2 mL, 13.0 mmol) at 0 °C. After 15 min, tert-butyl N-[(1E)-{[(tert-butoxy)carbonyl]amino}(1H-pyrazol-1-yl)methylidene]carbamate (commercially available from Sigma Aldrich) (1.1 g, 3.58 mmol) was added at the same temperature and allowed to stir at RT for 16 h. N₂ gas was bubbled into the reaction mixture for 15 minutes then water was added to the crude product followed by stirring for 5 minutes. The resulting precipitate was filtered, washed with water (2 x 10 mL) and dried under high vacuum affording a white solid (800 mg, 71%).
M/z 354.2 [M+H]⁺

### (b) 3-(2-carbamimidamidoethyl)-1-[({4-[(4-carboxy-1,3-thiazol-5-yl)sulfamoyl]-2-cyanophenyl}carbamoyl)methyl]-1H-imidazol-3-ium

To a solution of tert- butyl 5-{N-[(4-methoxyphenyl)methyl]4-(2-chloroacetamido)-3-cyanobenzenesulfonamido}-1,3-thiazole-4-carboxylate (Example 18d) (200 mg, 0.34 mmol) in CH₃CN (5 mL) was added tert- butyl N- [(1Z)- {[(tert-butoxy)carbonyl]amino}({[2-(1H-imidazol-1-yl)ethyl]amino})methylidene]carbamate (Example 19a) (183 mg, 0.51 mmol). The resulting reaction mixture was heated to 90 °C for 6 h. The mixture was evaporated under reduced pressure. The crude material was triturated with diethyl ether (2 x 5 mL) and dried under high vacuum to obtain a brown solid (260 mg). TFA (1 mL) was added to this solid at 0 °C and the solution stirred at RT for 3 hours.

The volatile components were evaporated under reduced pressure. The residue was triturated with diethyl ether (2 x 2 mL), n-pentane (2 x 2 mL) and dried under high vacuum. The crude product was purified by prep. HPLC to afford a light brown solid (39 mg, 22% over 2 steps).
¹H NMR (500 MHz, DMSO-*d₆*): δ 13.6 (br s, 1 H), 11.75 (br s, 1 H), 9.71 (br s, 1 H), 9.39 (s, 1 H), 8.63 (d, *J* = 1.5 Hz, 1 H), 8.36 (s, 2 H), 8.06 (s, 1 H), 7.92-7.87 (m, 4 H), 7.76 (d, *J* = 1.5 Hz, 1 H), 7.35 (d, *J* = 8.5 Hz, 1 H), 4.36 (br s, 2 H), 3.66 (s, 2 H)
M/z 518.1 [M]⁺

### LC-MS Conditions:

Column: Acquity UPLC BEH C18 (50 mm x 2.1 mm, 1.7 µm);
Mobile phase: (A) 0.1% FA in water; Mobile phase: (B) 0.1% FA in ACN;
Gradient: Time (min)/ %B: 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: KROMOSIL-C18 (150*25), 10 u;
Mobile phase: (A) 0.05% FA in water: Mobile phase: (B) ACN
Gradient: (T/%B): 0/2, 2/2, 8/30, 8.1/98, 10/98, 10.1/2, 12/2;

Flow Rate: 25 mL/min;

### Example 20 3-[({4-[(4-carboxy-1,3-thiazol-5-yl)sulfamoyl]-2-fluorophenyl}carbamoyl)methyl]-1-[2-(dimethylamino)ethyl]imidazol-1-ium

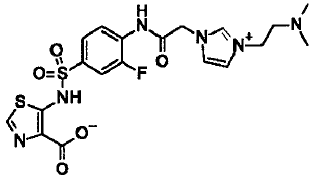

### (a) tert- butyl 5-{N-[(4-methoxyphenyl)methyl]3-fluoro-4-[2-(1H-imidazol-1-yl)acetamido]benzenesulfonamido}-1,3-thiazole-4-carboxylate

A mixture of Key Intermediate-1 (500 mg, 0.87 mmol) and imidazole (120 mg, 1.75 mmol) in acetonitrile (20 mL) was heated to 80 °C for 16 hours. The mixture was evaporated and the residue partitioned between ethyl acetate and water. The organic extract was dried and evaporated to afford a yellow solid (250 mg, 48%).
M/z 602.1 [M+1]⁺

### (b) 1-({[4-({4-[(tert-butoxy)carbonyl]-1,3-thiazol-5-yl}[(4methoxyphenyl)methyl]sulfamoyl)-2-fluorophenyl]carbamoyl}methyl)-3-[2-(dimethylamino)ethyl]-1H-imidazol-3-ium bromide

A mixture of tert- butyl 5-{N-[(4-methoxyphenyl)methyl]3-fluoro-4-[2-(1H-imidazol-1-yl)acetamido]benzenesulfonamido}-1,3-thiazole-4-carboxylate (Example 20a) (250 mg, 0.4 mmol), dimethylaminoethyl bromide hydrobromide salt (Sigma Aldrich) (120 mg, 2.07 mmol) and DIPEA (0.61 mL, 3.3 mmol) in acetonitrile (30 mL) was heated at 80 °C for 72 hours. The mixture was evaporated and the residue triturated with methanol-ether affording a brown solid (300 mg).

### (c) 3-[({4-[(4-carboxy-1,3-thiazol-5-yl)sulfamoyl]-2-fluorophenyl}carbamoyl)methyl]-1-[2-(dimethylamino)ethyl]imidazol-1-ium

A solution of 1-({[4-({4-[(tert-butoxy)carbonyl]-1,3-thiazol-5-yl}[(4methoxyphenyl)methyl]sulfamoyl)-2-fluorophenyl]carbamoyl}methyl)-3-[2-(dimethylamino)ethyl]-1H-imidazol-3-ium bromide (Example 20b) in TFA (20 mL) was stirred at room temperature for 4 hours then evaporated to dryness. The residue was triturated with ether then purified by prep. HPLC affording a white solid (70 mg).
¹H NMR (500 MHz, DMSO-*d₆*): δ 13.41 (br s, 1 H), 10.68 (br s, 1 H), 9.13 (s, 1 H), 8.08 (s, 1 H), 8.06 - 8.04 (m, 1 H), 7.76 (s, 1 H), 7.72 (s, 1 H), 7.54-7.43 (m, 2 H), 5.30 (s, 2 H), 4.31 (t, *J* = 5.5 Hz, 2 H), 2.63 (t, *J* = 5.5 Hz, 2 H), 2.17 (s, 6 H).
M/z 497.4 [M]⁺

### LC-MS Conditions:

Column: AQUITY UPLC BEH C18 (2.1 mm x 50 mm, 1.7 µm);
Mobile phase: (A) 0.1% FA in water; Mobile phase: (B) 0.1% FA in ACN
Gradient (T/%A): 0/97, 0.3/97, 2.7/2, 3.5/2, 3.51/97;
Flow: 0.6 mL/min;
Temp: 35 °C.

### Prep. HPLC Conditions:

Column: - X-SELECT-CN-(150*25); 10 u;
Mobile phase: (A): 0.05% FA in water: Mobile phase: (B) ACN
Gradient: (T%B): 0/5, 2/5, 5/20, 12/20, 12.1/98, 14/98, 14.1/5, 17/5;
Flow Rate: 20 mL/min;

### Example 21 3-[({4-[(4-carboxy-1,3-thiazol-5-yl)sulfamoyl]-2-fluorophenyl}carbamoyl)methyl]-1-[2-(trimethylammonio)ethyl]imidazol-1-ium

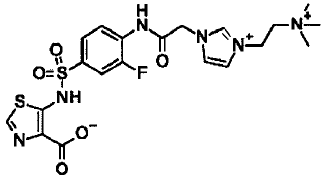

A solution of 1-({[4-({4-[(tert-butoxy)carbonyl]-1,3-thiazol-5-yl}[(4methoxyphenyl)methyl]sulfamoyl)-2-fluorophenyl]carbamoyl}methyl)-3-[2-(dimethylamino)ethyl]-1H-imidazol-3-ium bromide (Example 20b) (500 mg) in acetonitrile (10 mL) was treated with methyl iodide (310 mg, 2.2 mmol) and the mixture was stirred for 16 hours at RT. The mixture was evaporated and the residue (1g) dissolved in TFA (10 mL) and stirred for 16 hours then evaporated. The residue was triturated with ether then purified by prep. HPLC to afford a white solid (45 mg).
¹H NMR (500 MHz, DMSO-*d₆*): δ 13.45 (br s, 1 H), 11.03 (br s, 1 H), 9.34 (s, 1 H), 8.07 (s, 1 H), 7.99 (dd, *J* = 8.5 Hz, *J* = 7.5 Hz, 1 H), 7.85 (s, 1 H), 7.82 (s, 1 H), 7.50-7.45 (m, 2 H), 5.26 (s, 2 H), 4.81 (t, *J* = 6.5 Hz, 2 H), 3.89 (t, *J* = 6.5 Hz, 2 H), 3.12 (s, 9 H)
M/z 256.1 [M/2]⁺

### LC-MS Conditions:

Column: Acquity UPLC BEH C18 (50 mm x 2.1 mm, 1.7 um);
Mobile phase: (A) 0.1% FA in water; Mobile phase: (B) 0.1% FA in ACN
Gradient: Time (min)/ %B: 0/2, 0.2/2, 1.5/98, 2.6/98, 2.61/2, 3.2/2;
Column temperature: 45 °C;
Flow rate: 0.8 mL/min.

### Prep. HPLC Conditions:

Column: PRONTOSIL-C18 (250*20 mm), 10 um;
Mobile phase: (A) 0.05% FA in water: Mobile phase: (B) ACN;
Gradient: (T/%B):- 0/5, 2/5, 8/40, 9/40, 9.1/98, 11/98, 11.1/5, 14/5;
Flow Rate: 20 mL/min;

### Example 22 4-[({4-[(4-carboxy-1,3-thiazol-5-yl)sulfamoyl]-2-fluorophenyl}carbamoyl)methyl]-1-methyl-1,2,4-triazol-1-ium

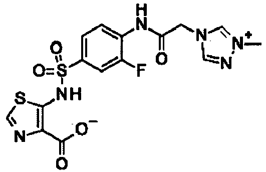

This compound was prepared by the same procedure as Example 1 with the exception that 1-methyl-1,2,4-triazole was used in place of 1-methylimidazole, affording the product as an off-white solid (132 mg, 42% over two steps).
¹H NMR (500 MHz, DMSO-*d₆*): δ 13.41 (br s, 1 H), 10.69 (s, 1 H), 10.03 (s, 1 H), 9.15 (s, 1 H), 8.08 (s, 1 H), 8.07-8.06 (m, 1 H), 7.56-7.53 (m, 2 H), 5.38 (s, 2 H), 4.13 (s, 3 H).
M/z 441.0 [M]⁺

### LC-MS Conditions:

Acquity UPLC BEH C18 (50 mm x 2.1 mm, 1.7 µm);
Mobile phase: A: 0.1% FA in water; Mobile phase: (B) 0.1% FA in ACN
Gradient: Time (min)/ %B: 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: - X-BRIDGE C18 (150 x 30 mm); 5 u;
Mobile phase: (A) 0.05 % TFA in water; Mobile phase: (B) ACN
Gradient (T/%B): 0/5, 8/40, 8.1/98, 10/98, 10.1/5, 13/5;

Flow Rate: 25 mL/min;

### Example 23 1-[({4-[(4-carboxy-1,3-thiazol-5-yl)sulfamoyl]-2,6-difluorophenyl}carbamoyl)methyl]-3-methyl-1H-1,2,3-triazol-3-ium

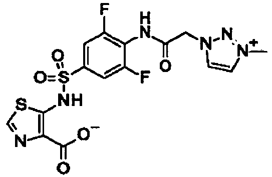

This compound was using the same procedures as for Example 9 except that the starting material used was Key Intermediate-2, affording the title compound as an off-white solid (40 mg, 16% over 2 steps).
¹H NMR (500 MHz, DMSO-*d₆*): δ 13.19 (br s, 1 H), 10.71 (s, 1 H), 8.91 (d, *J* = 1.0 Hz, 1 H), 8.86 (d, *J* = 1.0 Hz, 1 H), 8.14 (s, 1 H), 7.46 (d, *J* = 7.0 Hz, 2 H), 5.83 (s, 2 H), 4.36 (s, 3 H).
M/z 459.1 [M]⁺,

### LC-MS Conditions:

Column: Acquity UPLC BEH C18 (50 mm x 2.1 mm, 1.7 µm);
Mobile phase: (A) 0.1% FA in water; Mobile phase: (B) 0.1% FA in ACN
Gradient: Time (min)/ %B: 0/2, 0.4/2, 2.2/98, 2.6/98, 2.61/2, 3.0/2;
Column Temp: 60 °C;
Flow Rate: 0.8 mL/min.

### Prep. HPLC Conditions:

Column: SUNFIRE -C18 (150*30), 5 u;
Mobile phase: (A) 0.05 % FA in water; Mobile phase: (B) ACN
Gradient: (T/%B):- 0/5, 8/35, 9/35, 9.1/98, 11/98, 11.1/5, 14/5;
Flow Rate: 25 mL/min;

### Example 24 1-[({4-[(4-carboxy-1,3-thiazol-5-yl)sulfamoyl]-2-fluorophenyl}carbamoyl)methyl]-3-ethyl-1H-1,2,3-triazol-3-ium

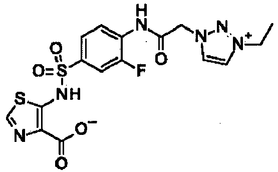

1-ethyl-1H-1,2,3- triazole

To a stirred solution of 1H-1,2,3-triazole (1.0 g, 14.4 mmol) in DMF (5.0 mL) was added K₂CO₃ (2.7 g, 17.3 mmol) at RT. Ethyl iodide (1.9 g, 14.4 mmol) was added to the reaction mixture at -10 °C and stirred at 0°C for 6 h. The reaction mixture was filtered and washed with ethyl acetate. The organic layer was concentrated to obtain a pale yellow oil (1.0g).
M/z 98.1 [M+H]⁺

1-[({4-[(4-carboxy-1,3-thiazol-5-yl)sulfamoyl]-2-fluorophenyl}carbamoyl)methyl]-3-ethyl-1H-1,2,3-triazol-3-ium This was prepared using the same procedures are Example 1 with the exception that 1-ethyl-1H-1,2,3- triazole was used in place of 1-methylimidazole, affording the title compound as a white solid (18 mg).
¹H NMR (500 MHz, DMSO-*d₆*): δ 13.40 (s, 1 H), 10.74 (s, 1 H), 8.96 (d, *J* = 1.0 Hz, 1 H), 8.92 (d, *J* = 1.0 Hz, 1 H), 8.08 (s, 1 H), 8.07-8.03 (m, 1 H), 7.56-7.54 (m, 2 H), 5.78 (s, 2 H), 4.71 (q, *J* = 7.5 Hz, 2 H), 1.54 (t, *J* = 7.5 Hz, 3 H).
M/z 455.0 [M]⁺

### LC-MS Conditions:

Column: Acquity UPLC BEH C18 (50 mm x 2.1 mm, 1.7 µm);
Mobile phase: (A) 0.1% FA in water; Mobile phase: (B) 0.1% FA in ACN
Gradient: Time (min)/ %A: 0.0/2, 0.5/2, 2.0/40, 5.0/98, 6.0/98, 7.0/2;
Column Temp: 60 °C;
Flow Rate: 0.5 mL/min.

### Prep. HPLC Conditions:

Column: YMC-TRIART-C18 (150*25), 10 u;
Mobile phase: (A) 0.05 % FA in water; Mobile phase: (B) ACN
Gradient: (T/%B):- 0/10, 8/35, 8.1/98, 10/98, 10.1/10, 12/10;
Flow Rate: 25 mL/min;

### Example 25 1-[({4-[(4-carboxy-1,3-thiazol-5-yl)sulfamoyl]-2-fluorophenyl}carbamoyl)methyl]-4-methyl-1H-1,2,4-triazol-4-ium

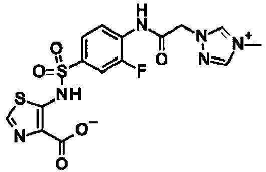

This compound was prepared from Key Intermediate 1 by the same procedure as for Example 1 except that 4-methyl-4H-1,2,4-triazole was used in place of 1-methylimidazole, affording the title compound as a white solid (100 mg, 43% over 2 steps).
¹H NMR (500 MHz, DMSO-*d₆*): δ 13.40 (br s, 1 H), 10.65 (s, 1 H), 10.10 (s, 1 H), 9.18 (s, 1 H), 8.08 (s, 1 H), 8.05 (dd, *J* = 8.5 Hz, *J* = 7.5 Hz, 1 H), 7.56-7.54 (m, 2 H), 5.53 (s, 2 H), 3.96 (s, 3 H).
M/z 441.0 [M]⁺

### LC-MS Conditions:

Acquity UPLC BEH C18 (50 mm x 2.1 mm, 1.7 µm);
Mobile phase: (A) 0.1% FA in ACN; Mobile phase: (B) 0.1% FA in water
Gradient: Time (min)/ %A: 0.0/2, 0.5/2, 2.0/40, 5.0/98, 6.0/98, 7.0/2;
Column Temp: 60 °C;
Flow Rate: 0.5 mL/min.

### Prep. HPLC Conditions:

Column: - X-SELECT-C18 (150*30), 5 u;
Mobile phase: 0.05% FA in water; ACN
Gradient (T/%B):- 0/5, 1/5, 8/40, 9/40, 9.1/98, 11/98, 11.1/5, 14/5;
Flow Rate: 25 mL/min;

### Example 26 5-amino-1-[({4-[(4-carboxy-1,3-thiazol-5-yl)sulfamoyl]-2-fluorophenyl}carbamoyl)methyl]-4-methyl-1H-1,2,3,4-tetrazol-4-ium

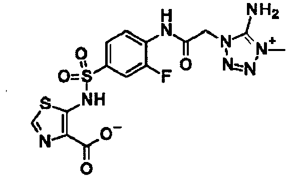

This compound was prepared from Key Intermediate 1 by the same procedure as for Example 1 except that 1-methyl-1H-1,2,3,4-tetrazol-5-amine was used in place of 1-methylimidazole, affording the title compound as a white solid (103 mg, 43% over 2 steps).
¹H NMR (500 MHz, DMSO-*d₆*): δ 13.22 (br s, 1 H), 10.67 (s, 1 H), 9.35 (s, 2 H), 8.12 (s, 1 H), 8.11 -8.07 (m, 1 H), 7.58-7.55 (m, 2 H), 5.40 (s, 2 H), 3.93 (s, 3 H)
M/z 457.0 [M]⁺

### LC-MS Conditions:

Acquity UPLC BEH C18 (50 mm x 2.1 mm, 1.7 µm);
Mobile phase: (A) 0.1% FA in water; Mobile phase: (B) 0.1% FA in ACN
Gradient: Time (min)/ %A: 0.0/2, 0.5/2, 2.0/40, 5.0/98, 6.0/98, 7.0/2;
Column Temp: 60 °C;
Flow Rate: 0.5 mL/min.

### Prep. HPLC Conditions:

Column:- SYMMETRY-C8 (300*19), 7u;
Mobile phase: (A) 0.1 % FA in water: Mobile phase: (B) ACN
Gradient (T/%B): 0/10, 8/75, 10/80, 10/98, 12/98, 12.1/10, 15/10;
Flow Rate: 25 mL/min;

### Example 27: In vitro enzyme inhibition assays and Antimicrobial susceptibility testing

**In vitro enzyme inhibition assays:** Enzyme inhibition assays are performed using purified MBL enzymes (NDM-1; VIM-1; VIM-2; IMP-1) in 10mM HEPES buffer pH 7.5 in 96-well microtiter plates. Imipenem (300µM) is used as substrates and its hydrolysis is followed at UV 299 nm during 10mn every 30 seconds using a Perkin Elmer Envision UV fluorescence plate reader. Hydrolysis rate data in presence of a range of inhibitors are analysed using Dotmatics database software and calculated IC50 values are converted to Ki values using the Cheng-Prusoff equation. Ki = IC50 / (1+([S]/Km) and where the Km values for NDM-1, VIM-2 and IMP-1 are 70 µM, 1.5 µM, 9 µM and 25 µM respectively. Compound dilution is performed in DMSO. Mean Ki values from multiple experiments are presented. Results are shown in Table 1.

**Antimicrobial susceptibility testing:** The operation is carried out using the 'broth micro-dilution method' according to the protocols M07-A8 established by the Clinical Laboratory Standards Institute (CLSI). Serial dilutions of the β-lactam antibiotic (Meropenem) are prepared in 96-well plates in cation-adjusted Mueller-Hinton broth (CAMHB); the concentration range is defined from 0.03 mg/L to 512 mg/L. The compounds are added at a constant concentration of 8 µg/mL. A bacterial inoculum of each strain (clinical isolates) is adjusted to a 0.5 McFarland turbidity standard in physiologic serum (0.9 % NaCl), then diluted 1:100 in CAMHB and added to each well to give a final bacterial cell number of 5x105 CFU/well. After incubation for 18-20 hours in a heating chamber at 37°C, the growth inhibition is evaluated by the absence of any bacterial development. The minimal inhibitory concentrations (MIC) are taken as the lowest concentration of antibiotic at which the test organism did not show visible growth; results are confirmed by measuring the optical density (OD) at 600 nm in a spectrophotometer.

### Antibiotic activity of β-lactam antibiotics on MBL expressing bacteria in the presence of the compounds of the invention

The compounds of Table 2 are added at a constant concentration of 8 µg/mL. The clinical strains used in these potentiation experiments are NTBC020 (E. coli strain expressing NDM-1, TEM-1 and CTX-M-15); NTBC035-2 (*K. pneumoniae* strain expressing NDM-1, CMY-4 and SHV-11); NTBC104-1 (*K. pneumoniae* strain expressing NDM-1 and SHV-11); NTBC123 (*K. pneumoniae* strain expressing NDM-1); NTBC018 (*C. freundii* strain expressing VIM-2); NTBC024 (*K. pneumoniae* strain expressing VIM-19, TEM-1 and CTX-M-3); NTBC042 (*E. coli* strain expressing VIM-1, TEM-1 , CTX-M-15, SHV-12); NTBC055 (E. Coli strain expressing VIM-1); NTBC062 *(K. pneumoniae* strain expressing IMP-1 and TEM-1) and NTBC039 (*K. oxytoca* strain expressing IMP-28).
For NDM-1 the Ki values of < 0.05 µM are designated (A); Ki values of 0.05 - 0.2 µM are designated (B); Ki values of 0.2 - 0.4 µM are designated (C).
For VIM-1 the Ki values of < 0.2 µM are designated (A); Ki values of 0.2 - 0.5 µM are designated (B); Ki values of 0.5 - 0.8 µM are designated (C).
For VIM-2 the Ki values of < 0.02 µM are designated (A); Ki values of 0.02 - 0.05 µM are designated (B); Ki values of 0.05 - 0.2 µM are designated (C).
For IMP-1 the Ki values of < 0.5 µM are designated (A); Ki values of 0.5 - 1 µM are designated (B); Ki values of 1 - 8 µM are designated (C).

MIC values of < 1 µg/mL are designated (A); MIC values of 1 - 2 µg/mL are designated (B);
MIC values of 2 - 128 µg/mL are designated (C).

**Table 1. Ki results**

| **Example** | **Ki (µM)** | | | |
|---|---|---|---|---|
| | **NDM-1** | **VIM-1** | **VIM-2** | **IMP-1** |
| 1 | (A) | (B) | (C) | (C) |
| 2 | (B) | (B) | (C) | (C) |
| 3 | (B) | (B) | (C) | (C) |
| 4 | (B) | (B) | | (C) |
| 5 | (B) | (C) | (C) | (C) |
| 6 | (C) | (C) | (C) | (C) |
| 7 | (C) | (A) | (B) | (C) |
| 8 | (B) | (B) | | (C) |
| 9 | (A) | (B) | (C) | (C) |
| 10 | (B) | (B) | | (C) |
| 11 | (A) | (A) | (B) | (C) |
| 12 | (A) | (A) | (C) | (C) |
| 13 | (B) | (C) | (C) | (C) |
| 14 | (A) | (A) | (B) | (C) |
| 15 | (A) | (A) | (B) | (C) |
| 16 | (A) | (A) | (A) | (C) |
| 17 | (A) | (A) | (A) | (C) |
| 18 | (B) | (A) | (A) | (B) |
| 19 | (C) | (A) | (B) | (C) |
| 20 | (A) | (A) | (C) | (C) |
| 21 | (A) | (A) | (A) | (C) |
| 22 | (B) | (B) | (C) | (B) |
| 23 | (B) | (B) | (C) | (C) |
| 24 | (B) | (B) | (C) | (B) |
| 25 | (B) | (B) | (C) | (C) |
| 26 | (A) | (B) | (C) | (B) |

**Table 2. MIC results**

| **Example** | **Strain** | | | | | | | | | |
|---|---|---|---|---|---|---|---|---|---|---|
| | **NTBC020** | **NTBC035-2** | **NTBC104-1** | **NTBC123** | **NTBC018** | **NTBC024** | **NTBC042** | **NTBC055** | **NTBC062** | **NTBC039** |
| 1 | (B) | (B) | (C) | (C) | (A) | (C) | (B) | (B) | (B) | (B) |
| 2 | (B) | (B) | (B) | (C) | (A) | (B) | (A) | (B) | (B) | (B) |
| 3 | (B) | (B) | (B) | (C) | (A) | (B) | (A) | (B) | (B) | (B) |
| 4 | (C) | (C) | (C) | (C) | (A) | (B) | (B) | (B) | (B) | (B) |
| 5 | N/A | N/A | N/A | N/A | N/A | N/A | N/A | N/A | N/A | N/A |
| 6 | (C) | (B) | (C) | (C) | (B) | (C) | (B) | (B) | (C) | (C) |
| 7 | (C) | (B) | (B) | (C) | (A) | (C) | (B) | (B) | (B) | (C) |
| 8 | (C) | (C) | (C) | (C) | (A) | (C) | (B) | (B) | (B) | (B) |
| 9 | (C) | (B) | (B) | (C) | (A) | (C) | (B) | (B) | (B) | (C) |
| 10 | (B) | (B) | (C) | (C) | (A) | (B) | (B) | (C) | (B) | (C) |
| 11 | (B) | (B) | (C) | (C) | (A) | (C) | (B) | (B) | (B) | (C) |
| 12 | (C) | (B) | (C) | (C) | (A) | (C) | (B) | (B) | (B) | (B) |
| 13 | (B) | (B) | (B) | (C) | (A) | (B) | (B) | (B) | (B) | (B) |
| 14 | (C) | (B) | (C) | (C) | (A) | (B) | (B) | (B) | (B) | (C) |
| 15 | (C) | (C) | (C) | (C) | (B) | (C) | (B) | (B) | (B) | (C) |
| 16 | (B) | (B) | (C) | (C) | (A) | (B) | (B) | (B) | (B) | (C) |
| 17 | (C) | (B) | (C) | (C) | (A) | (B) | d(B) | (B) | (B) | (C) |
| 18 | (C) | (C) | (C) | (C) | (A) | (C) | (B) | (B) | (B) | (B) |
| 19 | (C) | (C) | (C) | (C) | (B) | (B) | (B) | (B) | (C) | (C) |
| 20 | (B) | (B) | (C) | (C) | (B) | (B) | (B) | (B) | (B) | (C) |
| 21 | (C) | (C) | (B) | (C) | (B) | (C) | (B) | (B) | (B) | (B) |
| 22 | (C) | (A) | (C) | (C) | (B) | (C) | (B) | (B) | (B) | (C) |
| 23 | (C) | (B) | (C) | (C) | (A) | (C) | (B) | (B) | (B) | (C) |
| 24 | (B) | (B) | (C) | (C) | (A) | (B) | (B) | (B) | (B) | (B) |
| 25 | (B) | (B) | (B) | (C) | (A) | (B) | (A) | (B) | (B) | (B) |
| 26 | (C) | (B) | (C) | (C) | (B) | (B) | (B) | (B) | (B) | (C) |

## Claims

1. A compound which is a thiazole derivative of Formula (I), or a pharmaceutically acceptable salt thereof, 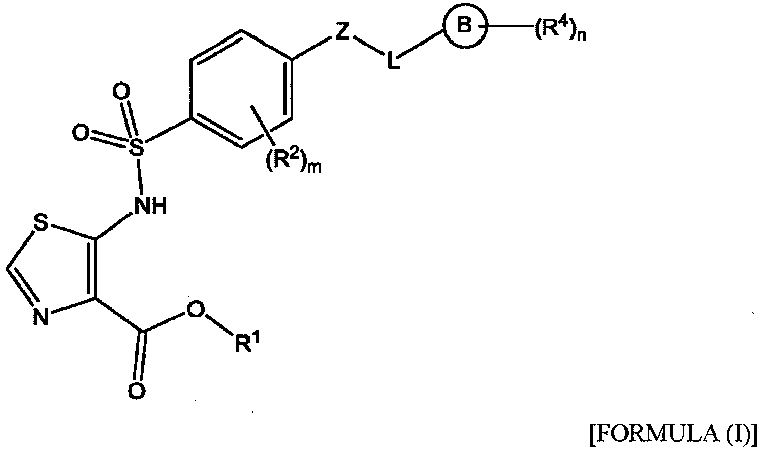 wherein
∘ R¹ is selected from:
• H, R^{1a} and -CH₂OC(O)R^{1a}, wherein R^{1a} is selected from an unsubstituted C₁ to C₄ alkyl group and phenyl; or
• where the compound of Formula (I) contains a positively charged nitrogen atom, R¹ may be absent, such that a COO⁻ group is present and the compound forms a zwitterion;
∘ each R² is independently selected from halo, R⁸, C₁₋₃ alkyl, O(C₁₋₃ alkyl) and S(C₁₋₃ alkyl), wherein the C₁₋₃ alkyl, O(C₁₋₃ alkyl) and S(C₁₋₃ alkyl) groups are unsubstituted or substituted with 1, 2 or 3 halo substituents and/or one R⁸ substituent; wherein each R⁸ is independently selected from CN and OH;
∘ *m* is 0, 1, 2 or 3
∘ *Z* is selected from -NR¹⁰C(O)-, -C(O)NR¹⁰-, -NR¹⁰C(O)NR¹¹-, -NR¹⁰C(O)O- and -OC(O)NR¹⁰;
∘ L is selected from C₁₋₄ alkylene, C₃₋₄ alkenylene, C₃₋₄ alkynylene, wherein L is unsubstituted or is substituted with 1 or 2 substituents selected from halogen, -OR¹⁰, -NR¹⁰R¹¹ and -N⁺R¹⁰R¹¹R¹²;
∘ Ring B is a five-membered heteroaromatic ring containing 1, 2, 3 or 4 nitrogen atoms, wherein ring B is joined to L via a ring nitrogen atom;
∘ each R⁴ is independently selected from
(i) halo or R⁵; or
(ii) C₁₋₄ alkyl, O(C₁₋₄ alkyl), S(C₁₋₄ alkyl), SO(C₁₋₄ alkyl) or SO₂(C₁₋₄ alkyl), each of which is unsubstituted or is substituted with 1, 2 or 3 halo substituents and/or one R⁵ substituent, or
(iii) two R⁴ groups together with the ring B atoms to which they are joined form a 5- or 6-membered carbocyclic or heterocyclic ring;
wherein
• each R⁵ is independently selected from CN, OH, -C(O)NR¹⁰R¹¹, -NR¹⁰R¹¹, -N⁺R¹⁰R¹¹R¹², -NR¹⁰C(NR¹¹)R¹², -C(NR¹⁰)NR¹¹R¹², and -NR¹⁰C(NR¹¹)NR¹²R¹³;
∘ *n* is 0, 1, 2, 3 or 4; and
∘ each R¹⁰, R¹¹, R¹² and R¹³ is independently H or unsubstituted C₁₋₂ alkyl.

2. A compound according to claim 1, wherein R¹ is H or unsubstituted C₁ to C₄ alkyl, preferably H; or, where the compound of Formula (I) contains a positively charged nitrogen atom, R¹ may be absent, such that a COO⁻ group is present and the compound forms a zwitterion.

3. A compound according to claim 1 or claim 2, wherein each R² is independently selected from halo, CN and C₁₋₃ alkyl, wherein the C₁₋₃ alkyl group is optionally substituted with 1, 2 or 3 halo substituents; and/or wherein m is 1 or 2.

4. A compound according to any one of the preceding claims, wherein Z is -NR¹⁰C(O)-, wherein R¹⁰ is H or unsubstituted C₁₋₂ alkyl, preferably R¹⁰ is H.

5. A compound according to any one of the preceding claims, wherein L is selected from unsubstituted C₁₋₄ alkylene, preferably L is -CH₂-.

6. A compound according to any one of the preceding claims, wherein Ring B is selected from imidazole, pyrazole, triazole and tetrazole.

7. A compound according to any one of the preceding claims, wherein
(i) each R⁴ is independently selected from halo, R⁵, unsubstituted O(C₁₋₄ alkyl), unsubstituted S(C₁₋₄ alkyl) and C₁₋₄ alkyl which is unsubstituted or substituted with 1, 2 or 3 halo substituents and/or one R⁵ substituent; wherein each R⁵ is independently selected from -NR¹⁰R¹¹, -N⁺R¹⁰R¹¹R¹², -NR¹⁰C(NR¹¹)R¹², -C(NR¹⁰)NR¹¹R¹², and -NR¹⁰C(CN¹¹)NR¹²R¹³; or two R⁴ groups together with the ring B atoms to which they are joined form a 5- or 6-membered heterocyclic ring; and/or
(ii) n is 1 or 2.

8. A compound according to any one of the preceding claims, wherein Ring B is substituted with one group R⁴ which is selected from R⁵, unsubstituted O(C₁₋₄ alkyl), unsubstituted S(C₁₋₄ alkyl) and C₁₋₄ alkyl which is unsubstituted or substituted with one R⁵ substituent; wherein R⁵ is independently selected from -NR¹⁰R¹¹, -N⁺R¹⁰R¹¹R¹² and -NR¹⁰C(NR¹¹)NR¹², wherein each R¹⁰, R¹¹, R¹² and R¹³ is independently H or methyl; and Ring B is optionally further substituted with a methyl group;
wherein at least one of the substituents on Ring B is on a ring nitrogen atom.

9. A compound according to any one of the preceding claims, wherein:
- R¹ is H, or, where the compound of Formula (I) contains a positively charged nitrogen atom, R¹ may be absent, such that a COO⁻ group is present and the compound forms a zwitterion;
- each R² is independently selected from F and CN;
- m is 1 or 2;
- -Z-L- is -NHC(O)-(CH₂)-;
- Ring B is selected from imidazole, pyrazole, triazole and tetrazole;
- *n* is 1 or 2;
- one group R⁴ is selected from R⁵, unsubstituted O(C₁₋₄ alkyl), unsubstituted S(C₁₋₄ alkyl) and C₁₋₄ alkyl which is unsubstituted or substituted with one R⁵ substituent; wherein R⁵ is independently selected from -NR¹⁰R¹¹, -N⁺R¹⁰R¹¹R¹² and -NR¹⁰C(NR¹¹)NR¹²; and, where present, the other group R⁴ is methyl; and
- each R¹⁰, R¹¹, R¹² and R¹³ is independently H or methyl.

10. A compound according to claim 1, which is
1-[({4-[(4-carboxy-1,3-thiazol-5-yl)sulfamoyl]-2-fluorophenyl}carbamoyl)methyl]-3-methyl-1H-imidazol-3-ium;
1-[({4-[(4-carboxy-1,3-thiazol-5-yl)sulfamoyl]-2-fluorophenyl}carbamoyl)methyl]- 2,3-dimethyl-1H-imidazol-3-ium;
2-amino-1-[({4-[(4-carboxy-1,3-thiazol-5-yl)sulfamoyl]-2-fluorophenyl}carbamoyl)methyl]-3-methyl-1H-imidazol-3-ium;
1-[({4-[(4-carboxy-1,3-thiazol-5-yl)sulfamoyl]-2-fluorophenyl}carbamoyl)methyl]-3-methyl-2-(methylsulfanyl)-1H-imidazol-3-ium;
1-[({4-[(4-carboxy-1,3-thiazol-5-yl)sulfamoyl]-2-fluorophenyl}carbamoyl)methyl]-1H,5H,6H,8H-4*lambda*5-imidazo[2,1-c][1,4]oxazin-4-ylium;
1-[({4-[(4-carboxy-1,3-thiazol-5-yl)sulfamoyl]-2-fluorophenyl}carbamoyl)methyl]-3,5-dimethyl-1H-imidazol-3-ium;
1-[({4-[(4-carboxy-1,3-thiazol-5-yl)sulfamoyl]-2-fluorophenyl}carbamoyl)methyl]-3,4-dimethyl-1H-imidazol-3-ium;
3-amino-1-[({4-[(4-carboxy-1,3-thiazol-5-yl)sulfamoyl]-2-fluorophenyl}carbamoyl)methyl]-2-me1hyl-1H-pyrazol-2-ium;
1-[({4-[(4-carboxy-1,3-thiazol-5-yl)sulfamoyl]-2-fluorophenyl}carbamoyl)methyl]-3-methyl-1H-1,2,3-triazol-3-ium;
1-[({4-[(4-carboxy-1,3-thiazol-5-yl)sulfamoyl]-2-fluorophenyllcarbamoyl)methyl]-2-methyl-1H-pyrazol-2-ium;
3-(2-aminoethyl)-1-[({4-[(4-carboxy-1,3-thiazol-5-yl)sulfamoyl]-2-fluorophenyl}carbamoyl)methyl]-1H-imidazol-3-ium;
3-(2-carbamimidamidoethyl)-1-[({4-[(4-carboxy-1,3-thiazol-5-yl)sulfamoyl]-2-fluorophenyl}carbamoyl)methyl]-1H-imidazol-3-ium;
1-[({4-[(4-carboxy-1,3-thiazol-5-yl)sulfamoyl]-2,6-difluorophenyl}carbamoyl)methyl]-3-methyl-1H-imidazol-3-ium;
3-(2-aminoethyl)-1-[({4-[(4-carboxy-1,3-thiazol-5-yl)sulfamoyl]-2,6-difluorophenyl}carbamoyl)methyl]-1H-imidazol-3-ium;
1-(2-carbamimidamidoethyl)-3-[({4-[(4-carboxy-1,3-thiazol-5-yl)sulfamoyl]-2,6-difluorophenyl}carbamoyl)methyl]imidazol-1-ium;
3-(3-aminopropyl)-1-[({4-[(4-carboxy-1,3-thiazol-5-yl)sulfamoyl]-2-fluorophenyl}carbamoyl)methyl]-1H-imidazol-3-ium;
3-(3-carbamimidamidopropyl)-1-[({4-[(4-carboxy-1,3-thiazol-5-yl)sulfamoyl]-2-fluorophenyl}carbamoyl)methyl]-1H-imidazol-3-ium;
3-(2-aminoethyl)-1-[({4-[(4-carboxy-1,3-thiazol-5-yl)sulfamoyl]-2-cyanophenyl}carbamoyl)methyl]-1H-imidazol-3-ium;
3-(2-carbamimidamidoethyl)-1-[({4-[(4-carboxy-1,3-thiazol-5-yl)sulfamoyl]-2-cyanophenyl}carbamoyl)methyl]-1H-imidazol-3-ium;
3-[({4-[(4-carboxy-1,3-thiazol-5-yl)sulfamoyl]-2-fluorophenyl}carbamoyl)methyl]-1-[2-(dimethylamino)ethyl]imidazol-1-ium;
3-[({4-[(4-carboxy-1,3-thiazol-5-yl)sulfamoyl]-2-fluorophenyl}carbamoyl)methyl]-1-[2-(trimethylammonio)ethyl]imidazole-1-ium;
4-[({4-[(4-carboxy-1,3-thiazol-5-yl)sulfarnoyl]-2-fluorophenyl}carbamoyl)methyl]-1-methyl-1,2,4-triazol-1-ium;
1-[({4-[(4-carboxy-1,3-thiazol-5-yl)sulfamoyl]-2,6-difluorophenyl}carbamoyl)methyl]-3-methyl-1H-1,2,3-triazol-3-ium;
1-[({4-[(4-carboxy-1,3-thiazol-5-yl)sulfamoyl]-2-fluorophenyl}carbamoyl)methyl]-3-ethyl-1H-1,2,3-triazol-3-ium;
1-[({4-[(4-carboxy-1,3-thiazol-5-yl)sulfamoyl]-2-fluorophenyl}carbamoyl)methyl]-4-methyl-1H-1,2,4-triazol-4-ium;
5-amino-1-[({4-[(4-carboxy-1,3-thiazol-5-yl)sulfamoyl]-2-fluorophenyl}carbamoyl)methyl]-4-methyl-1H-1,2,3,4-tetrazol-4-ium;
or a pharmaceutically acceptable salt of one of these compounds.

11. A compound according to any one of claims 1 to 10 for use in the removal or reduction of antibiotic resistance in Gram-negative bacteria.

12. A compound according to any one of claims 1 to 10 for use in the treatment or prevention of bacterial infection.

13. A compound for use according to claim 12, which is for use in the treatment or prevention of bacterial infection by co-administration with an antibiotic agent and/or a serine-β-lactamase inhibitor.

14. A compound for use according to any one of claims 11 to 13 wherein the Gram-negative bacteria are selected from Enterobacteriaceae, Pseudomonadaceae and Moraxellaceae, or the bacterial infection is caused by bacteria selected from Enterobacteriaceae, Pseudomonadaceae and Moraxellaceae.

15. A compound for use according to claim 14 wherein the bacteria selected from Enterobacteriaceae, Pseudomonadaceae and Moraxellaceae are selected from *Klebsiella pneumonia*, *Escherichia coli, Pseudomonas aeruginosa*, *Burkholderia cepacia* and *Acinetobacter baumannii,* and/or the bacterial infection is caused by Carbapenem Resistant Enterobacteriaceae.
